(19) **Europäisches Patentamt / European Patent Office / Office européen des brevets**

(11) **EP 4 551 029 B1**

(12) **EUROPEAN PATENT SPECIFICATION**

(45) Date of publication and mention
of the grant of the patent:
**24.06.2026 Bulletin 2026/26**

(21) Application number: **23738031.6**

(22) Date of filing: **04.07.2023**

(51) International Patent Classification (IPC):
*A23C 1/00* *(2006.01)*    *A23C 9/142* *(2006.01)*
*A23C 1/12* *(2006.01)*    *C07H 1/08* *(2006.01)*
*C07H 3/04* *(2006.01)*    *C13K 5/00* *(2006.01)*

(52) Cooperative Patent Classification (CPC):
**C07H 1/08; A23C 1/12; A23C 9/1425;
A23C 9/1427; C07H 3/04; C13K 5/00;**
A23C 2210/20; A23C 2210/206

(86) International application number:
**PCT/EP2023/068355**

(87) International publication number:
**WO 2024/008701 (11.01.2024 Gazette 2024/02)**

(54) **METHOD OF REMOVING CALCIUM CITRATE FROM A LIQUID DAIRY STREAM**

VERFAHREN ZUM ENTFERNEN VON CALCIUMCITRAT AUS EINEM FLÜSSIGEN MILCHSTROM

PROCÉDÉ D'ÉLIMINATION DU CITRATE DE CALCIUM À PARTIR D'UN FLUX LAITIER LIQUIDE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **08.07.2022 EP 22183750**

(43) Date of publication of application:
**14.05.2025 Bulletin 2025/20**

(73) Proprietor: **Arla Foods amba
8260 Viby J (DK)**

(72) Inventors:
- **NIELSEN, Kenneth Dyrmose
8260 Viby J (DK)**
- **DAHLBERG, Anders
8260 Viby J (DK)**
- **ANDERSEN, Troels Møller
8260 Viby J (DK)**

- **JENSEN, Steen Lindegaard
8260 Viby J (DK)**
- **FRIIS, Christoffer
8260 Viby J (DK)**
- **BECH, Jens Kristian
8260 Viby J (DK)**
- **DAMBORG, Vinni Kragbæk
8260 Viby J (DK)**
- **NIELSEN, Niels Christian
8260 Viby J (DK)**

(74) Representative: **Plougmann Vingtoft a/s
Strandvejen 70
2900 Hellerup (DK)**

(56) References cited:
**EP-B1- 3 262 200    US-A- 4 202 909
US-A1- 2016 044 933**

**Description**

**Technical field of the invention**

The present invention relates to a method of removing calcium citrate from a liquid dairy stream. In particular, the present invention relates to a method of removing calcium citrate from a liquid dairy stream wherein the liquid dairy stream has a pH below 6.2 or is optionally adjusted to be below 6.2, and wherein said liquid dairy stream having a pH below 6.2 is subjected to a step of precipitation of calcium citrate. Subsequently, the precipitated calcium citrate can be separated.

**Background of the invention**

In the production of crystallized lactose from a dairy stream, such as a) a permeate from ultrafiltration of a dairy product or b) whey, the presence of minerals may precipitate during lactose production, or co-precipitate with lactose during the lactose crystallisation which results in obtaining a crystallized lactose product having more ash and a decreased value.

In relation to lactose production, calcium, citric acid and phosphor have a major impact on several of the steps in the lactose production:

- high levels of calcium, citric acid and phosphor can result in fouling of the membrane plants
- high levels of calcium, citric acid and phosphor can result in fouling of evaporators
- high levels of calcium, citric acid and phosphor can result in high levels of ash in the obtained lactose powder, since:

  ○ the calcium phosphates and citrates formed can act as nucleation for the lactose crystallisation process, and thereby be difficult to wash out of the lactose crystals in the washing step,
  ○ the calcium phosphate and citrate particles formed follow the decanter and centrifuge slurries during processing and are therefore not removed from the lactose crystals by the separation and washing processes.

The content of soluble calcium in dairy products is high and will typically be present along with anions such as citrate and phosphates. The presence of the free calcium and anions can result in the creation of calcium citrate and calcium phosphate precipitates, which can be problematic for efficient production of lactose crystals. Hence, a calcium phosphate precipitate is typically created and removed from the dairy stream used for preparing the lactose crystals in order to increase the quality of the lactose product. Calcium phosphate is typically removed from the dairy stream by increasing the pH of the dairy stream to above pH 7.0, followed by heating of the pH adjusted dairy stream to precipitate calcium phosphate that can be separated from the dairy stream before the lactose crystallization such that the quality of the lactose crystal product is increased.

WO 2016/135172 A1 discloses a method of separating calcium phosphate from a whey permeate by adjusting the pH to about 7.2 by addition of caustic $Mg(OH)_2$ or NaOH, after which the whey permeate is heated to about 80°C. The precipitated calcium phosphate is then removed by centrifugation or membrane filtration.

WO 2016/193138 A1 discloses a method of obtaining crystallised lactose from one or more aqueous solutions comprising lactose from whey or whey permeate. The method involves demineralising a lactose solution, whey or whey permeate, and the demineralisation is by nanofiltration. US 4 202 909 A discloses a method of removing calcium citrate from whey. EP 3 262 200 B1 discloses a method for removing calcium phosphates from whey.

Typically, it is avoided to add citric acid to a milk product if the streams/fractions obtained therefrom are used for lactose production. However, citric acid or citrate may be added to milk in connection with cheese production resulting in ultrafiltration permeates comprising citric acid/citrate and acid whey. Typically, such acid containing ultrafiltration permeates and acid whey are used as animal feed. Citric acid may be added to a dairy product in connection with preparing cheese to disassociate calcium bound in casein micelles into free calcium. The calcium and citric acid will form calcium citrate, and the presence of calcium citrate is problematic for efficient production of lactose crystals.

The inventors of the present invention have found that the methods known for removing calcium phosphate from a dairy stream are unsuitable for efficient removal of calcium citrate.

It is important to remove calcium phosphate and calcium citrate from a dairy stream, because calcium phosphate and calcium citrate can co-precipitate with lactose during the lactose crystallisation and therefore decrease the yield of lactose crystals. Further, the impurities in the lactose crystallised product become higher if calcium phosphate and calcium citrate are present.

In addition, problems with fouling of membranes or heating elements during concentration of dairy solutions are often observed if a dairy product is concentrated to a high solid content. Fouling of membranes and heating elements is problematic since it results in the need of cleaning more frequently.

Hence, a method of removing calcium citrate from a dairy stream would be advantageous, and in particular a method of efficiently removing calcium citrate without the need for improved cleaning of membranes and heating elements used for concentrating would be advantageous.

## Summary of the invention

Thus, an object of the present invention relates to providing a method of efficiently removing calcium citrate from a liquid dairy stream. In particular, the present invention relates to a method of efficiently removing calcium citrate from a dairy liquid stream with minimum fouling of membranes and heating elements.

In the production of lactose, there is often a type of decalcification step. The inventors of the present invention have surprisingly found that by first precipitating calcium citrate at low pH and subsequently precipitating calcium phosphate at higher pH is beneficial for the lactose production.

In particular, it is an object of the present invention to provide a method that solves the above mentioned problems of the prior art with not removing calcium citrate and with fouling of membranes and heating elements.

Thus, one aspect of the invention relates to a method of removing calcium citrate from a liquid dairy stream, wherein the method comprises the following steps:

> i) providing a liquid dairy stream comprising a) calcium and b) citric acid and/or citrate, and wherein the liquid dairy stream has a pH below 6.2;
> ii) concentrating the liquid dairy stream until the degrees Brix is in the range of 5°Bx to 24°Bx, wherein the concentrating is by using one of more or nanofiltration and reverse osmosis;
> iii) subjecting the concentrated liquid dairy stream to a precipitation step to precipitate calcium citrate, the precipitation step comprises either the precipitation in step A) or the precipitation in step B):
>
>> A) heating the concentrated liquid dairy stream to a temperature of 40°C to 85°C for at least 5 minutes to precipitate calcium citrate;
>> B) seeding the concentrated liquid dairy stream and storing the seeded concentrated liquid dairy stream at a temperature of 10°C to 40°C;
>
> iv) optionally cooling the heat-treated concentrated liquid dairy product;
> v) separating precipitated calcium citrate from the heat-treated liquid dairy stream by using one or more devices for mechanical separation of precipitated calcium citrate from the liquid dairy stream.

Another aspect of the invention relates to a method of preparing crystallized lactose, wherein said method comprises the following steps:

> A) providing the liquid dairy stream where calcium citrate has been removed according to the invention or the liquid dairy stream where calcium citrate and calcium phosphate have been removed according to the invention;
> B) adjusting the pH to be in the range of 5.5 to 6.2
> C) concentrating the pH adjusted liquid dairy stream of step B) until the degrees Brix is in the range of 55 to 65°Bx at a temperature of 50°C to 80°C;
> D) cooling to a temperature of 5°C to 20°C to obtain crystallized lactose and mother liquor;
> E) separating lactose crystals from the mother liquor;
> F) optionally washing the lactose crystals with water.

## Brief description of the figures

Figure 1 shows the index numbers for different nutrients for "UF permeate high calcium" versus "UF permeate low calcium"

Figure 2 shows the calcium content in percentage of the total solid content in different samples of "UF permeate high calcium" and "UF permeate low calcium" concentrated to different degrees Brix.

Figure 3 shows the content of phosphor in percentage of the total solid content in different samples of "UF permeate high calcium" and "UF permeate low calcium" concentrated to different degrees Brix.

Figure 4 shows the content of citric acid in percentage of total solid content in different samples of "UF permeate high calcium" and "UF permeate low calcium" concentrated to different degrees Brix.

Figure 5 shows flux data as a measurement of fouling trend for samples of "UF permeate high calcium" and "UF permeate low calcium" that have continuously been RO concentrated from 5°Bx to 16°Bx.

Figure 6 shows flux data as a measurement of fouling trend for samples of "UF permeate high calcium" and "UF permeate low calcium" that have continuously been RO concentrated from 16°Bx to 27°Bx.

Figure 7 shows the calcium content of the total solid content in samples of "UF permeate high calcium" that have been adjusted to different pH values followed by heating.

Figure 8 shows the citric acid content of the total solid content in samples of "UF permeate high calcium" that have been adjusted to different pH values followed by heating.

Figure 9 shows the phosphor content of the total solid content in samples of "UF permeate high calcium" that have been adjusted to different pH values followed by heating.

Figure 10 shows the calcium content of the total solid content in samples of "UF permeate low calcium" that have been adjusted to different pH values followed by heating.

Figure 11 shows the citric acid content of the total solid content in samples of "UF permeate low calcium" that have been adjusted to different pH values followed by heating.

Figure 12 shows the phosphor content of the total solid content in samples of "UF permeate low calcium" that have been adjusted to different pH values followed by heating.

Figure 13 shows the calculated solubility product in samples of "UF permeate high calcium" and "UF permeate low calcium" being adjusted to different pH values.

Figure 14 shows the calculated solubility product in samples of "UF permeate high calcium" and "UF permeate low calcium" being adjusted to different pH values.

Figure 15 shows the development in calcium and citric acid concentration per total solids in the supernatant fractions of "UF permeate high calcium" being seeded versus not being seeded over a period of time.

Figure 16 shows the heat transfer coefficient measured of the samples of "UF permeate high calcium" heated to different temperatures.

Figure 17 shows the pressure drop of the samples of "UF permeate high calcium" heated to different temperatures.

Figure 18 shows the calcium content of the total solid content in samples of "UF permeate high calcium" concentrated to different degrees Brix.

Figure 19 shows the citric acid content of the total solid content in samples of "UF permeate high calcium" concentrated to different degrees Brix.

Figure 20 shows the phosphor content of the total solid content in samples of "UF permeate high calcium" concentrated to different degrees Brix.

Figure 21 shows the content of calcium, phosphor and citric acid in samples of "UF permeate high calcium" concentrated from 5°Bx to 16°Bx and 27°Bx respectively, and calcium citrate is separated by use of ultrafiltration.

Figure 22 shows the content of calcium, phosphor and citric acid in samples of "UF permeate high calcium" concentrated from 5°Bx to 16°Bx and 27°Bx respectively, and calcium citrate is separated by use of clarifier.

Figure 23 shows the content of calcium, phosphor and citric acid in samples of "UF permeate high calcium" concentrated from 5°Bx to 16°Bx and 27°Bx respectively, and calcium citrate is separated by use of clarifier and ultrafiltration.

Figure 24 shows the flux data as a measurement of fouling trend for samples of "UF permeate high calcium" concentrated to 27°Bx, a reference UF permeate of milk (no citric acid added), and a UF permeate high calcium where calcium citrate has not been removed.

The present invention will now be described in more detail in the following.

**Detailed description of the invention**

Definitions:

Prior to discussing the present invention in further details, the following terms and conventions will first be defined:

All references to singular characteristics or limitations of the present invention shall include the corresponding plural characteristics or limitations, and vice versa, unless otherwise specified or clearly implied to the contrary by the context in which the reference is made.

The term "degrees Brix" refers to the content of soluble components of an aqueous solution. The degrees Brix may also be referred to as "°Bx". In the dairy industry, the degree of Brix is used as an indicator for the soluble dry matter content in ultrafiltration permeates. Soluble components all contribute to the measured degree of Brix, such as lactose, proteins, and minerals.

Hence, the measurement of "degrees Brix" is an alternative method of measuring dissolved solids in a liquid, i.e. the solid content in a liquid. 1°Bx is equal to about 1 gram of sugar (e.g. sucrose or lactose) in 100 gram of a solution and represents the strength of the solution as percentage by mass. However, in the liquid dairy stream used in the method of the present invention, other dissolved solids than pure lactose, including minerals and citric acid, is dissolved therein. Therefore, the °Bx is not equivalent to the solid content, but close to. In the context of the present invention, the degrees Brix have been measured to indicate the solid content, because the method of measuring °Bx is faster than the method of measuring the total solid content.

In the context of the present invention, 1°Bx corresponds to 0.9 to 1.0 gram of solids in 100 gram in the liquid dairy stream. In particular, 1°Bx corresponds to 0.92 to 0.99 gram solids in the liquid dairy stream.

Liquid dairy stream:

The liquid dairy stream is any liquid dairy stream that comprises calcium and citric acid and/or citrate and should not be limited to any specific stream.

However, in an embodiment of the invention, the liquid dairy stream is selected from the group consisting of whey, ultrafiltration permeate, microfiltration permeate, and whey protein concentrate.

The ultrafiltration permeate is typically a permeate stream obtained by ultrafiltration of a liquid dairy milk product. For example, the ultrafiltration permeate can be a permeate stream obtained by ultrafiltration of milk. The ultrafiltration permeate may also be the permeate obtained after ultrafiltration of whey in connection with production of cheese curds. Furthermore, the ultrafiltration permeate can be the permeate obtained after ultrafiltration of a milk that has been added, coagulation enzymes (for example rennet) and chemical acidifying agents (for example citric acid and lactic acid).

The microfiltration permeate is typically a permeate stream obtained by microfiltration of a liquid dairy milk product. For example, the microfiltration permeate can be a permeate obtained by microfiltration of milk, said microfiltration permeate may be referred to as a milk serum protein concentrate. In the context of the present invention, the terms "milk serum protein" or "serum protein" refer to the protein found in the milk serum. The milk serum proteins typically include beta-lactoglobulin, alpha-lactalbumin, bovine serum albumin, immunoglobulin and osteopontin as well as lactoferrin and lactoperoxidase. The milk serum protein may furthermore contain a significant amount of beta-casein when the milk feed has been stored at low temperature (4°C) without it being subsequently heat-treated.

The term "whey" refers to the liquid obtained after casein is precipitated and strained from the milk.

For example, precipitation of casein may be obtained by using a coagulation enzyme, for example rennet. In other methods known in the art, the coagulation is due to acidification or a combination of acidification and addition of coagulation enzymes. The whey obtained from precipitation of casein by use of a coagulation enzyme is typically referred to as sweet whey, and the whey obtained from acid precipitation of casein micelles is typically referred to as acid whey or sour whey. A whey protein concentrate is obtained by concentrating whey proteins.

Typically, acid whey has limited use, because of the acids present and hence the low pH. On the contrary, the sweet whey can be further processed into various products, e.g. whey protein products or lactose products. However, with the present invention it will also be possible to use acid whey for production of lactose products. The acid whey can be obtained both by chemical acidified whey and or by adding acid producing microorganisms.

The milk used for obtaining an ultrafiltration permeate and/or a microfiltration permeate may, for example, be whole milk, low-fat milk, reduced fat milk, fat-free milk, reconstituted milk powder, heat-treated milk (e.g. pasteurized milk, and UHT milk), raw unfiltered milk, homogenized milk, mineral reduced milk, whey protein reduced milk, and combinations thereof.

Preferably, the milk is pasteurized milk, and especially pasteurized bovine milk. When referring to pasteurized milk, it may in principle be any type of the above mentioned milk products that have been pasteurized, such as pasteurized whole milk, low-fat milk, reduced fat milk, fat-free milk, raw unfiltered milk, homogenized milk, mineral reduced milk, and whey protein reduced milk.

The milk that the ultrafiltration permeate and/or the microfiltration permeate is obtained from may be based on milk from mammals such as cows, buffalos, goats, sheep, yaks, pigs, horses, ewes, mares, or mixtures thereof. In a preferred embodiment of the present invention, the milk is from cows, i.e. bovine milk. The terms bovine milk and cow's milk refer to the same.

The pH of the liquid dairy stream should be below pH 6.2. If the pH of the liquid dairy stream is higher than 6.2, calcium citrate will only be removed in very small amounts. In a preferred embodiment of the present invention, the pH of the liquid dairy stream is in the range of 5.0 to 6.2. The pH of the liquid dairy stream should preferably be above 5.0, because a lower pH results in decreased removal of calcium citrate. Hence, if the pH of the liquid dairy stream is lower than 5.0, less calcium is precipitated as calcium citrate, and it is not desired to have such high amount of citrate present in the product when making a crystallised lactose product. In a further preferred embodiment of the invention, the pH of the liquid dairy stream is in the range of 5.2 to 6.1, such as in the range of 5.5 to 6.1. Most preferably, the pH of the liquid dairy stream is in the range of 5.6 to 6.0.

Furthermore, the pH of the liquid dairy stream may in an embodiment of the invention be adjusted to be in the range of 5.5 to 6.0. The pH adjustment is preferably after step i) and before step ii) in the method of removing calcium citrate according to the present invention. For example, if an ultrafiltration (UF) permeate is used that has a pH of 6.1, the pH of the liquid dairy product could be adjusted to be in the range of 5.5 to 6.0 in order to increase the formation of calcium citrate precipitates, and thereby, the efficiency in removing calcium citrate. Calcium citrate can be removed when the pH of the liquid dairy stream is in the range of 5.0 to 6.2. However, the calcium citrate is most efficiently removed when the pH is in the range of 5.5 to 6.1 and even more efficiently removed when the pH is the range of 5.7 to 6.0. The pH of the liquid dairy stream can be adjusted either by adding a base or an acid, it depends on whether the pH should be increased or decreased.

If an acid is added to adjust the pH, it is preferably a food grade acid, for example citric acid, lactic acid, acetic acid, hydrochloric acid, carbon dioxide, or glucono-delta-lactone. If the pH of the liquid dairy stream is low, the pH could be increased by adding a base, for example sodium hydroxide, calcium hydroxide, potassium hydroxide, or magnesium hydroxide.

Concentration of liquid dairy stream:

The liquid dairy stream is concentrated to a degrees Brix being in the range of 5°Bx to 24°Bx. It is preferred that the liquid dairy stream is concentrated to at least 5°Bx because an increased concentration results in a higher degree of supersaturation, a higher driving force towards precipitation and decreases the volume needed to be heated. However, the liquid dairy stream should not be concentrated to a degree of Brix above 24°Bx, because a higher concentration results in fouling of membranes and heating elements.

In an embodiment of the present invention, the liquid dairy stream is concentrated to a degrees Brix in the range of 8°Bx to 22°Bx, such as 10°Bx to 20°Bx, preferably 12°Bx to 18°Bx and even more preferably, 12°Bx to 16°Bx.

In the method of the invention, the concentration of the liquid dairy stream is one or more selected from the group consisting of reverse osmosis and nanofiltration. Hence, the concentration of the liquid dairy product is obtained by subjecting the liquid dairy stream to one or more of the means for concentration selected from the group consisting of reverse osmosis and nanofiltration. In the method of the present invention, evaporation is not used.

Most preferably, concentration is by using reverse osmosis. By using reverse osmosis, water is removed from the permeate, but the solid components are maintained in the retentate.

During concentration of an liquid dairy stream, the liquid dairy stream should preferably not be heated. Hence, evaporation using heating is not preferred, since evaporation at temperatures above 50°C will result in rapid precipitation of calcium citrate. It is not desired to have precipitated calcium citrate in the evaporator. Furthermore, vacuum evaporation is also not preferred. Vacuum evaporation may concentrate a liquid at lower temperatures than when using evaporation without vacuum. Even though the liquid can boil at lower temperatures, the temperature will still be too high to avoid precipitation of calcium citrate. As mentioned earlier, it is not desired to have precipitated calcium citrate in the concentration step. On the contrary, it is desired to control precipitation of calcium citrate, which can be done with the method of the present invention.

In the context of the present invention, the term "nanofiltration" (NF) refers to what is normally understod by nanofiltration by the skilled person. Hence, NF refers in the context of the present invention to seperation by using membrane filtration with a membrane having a molecular weight cut-off (MWCO) in the range of 20 to 1000 Dalton (Da), which equals that a molucular weight (MW) of a known substance (e.g. dextran or polyethylene glycol) is 90% retained by the membrane in the seperation step.

In an embodiment of the present invention, the MWCO of a NF membrane used is in the range of 100 to 800 Da, most preferably 200 to 400 Da.

In the context of the present invention, the term "reverse osmosis" (RO) refers to what is normally understod by reverse osmosis by the skilled person. Hence, RO refers in the context of the present invention to seperation of water from solids by using membrane filtration with a membrane having a molecular weight cut-off (MWCO) below 50 Da. The RO membrane will retain 95% to >99% sodium chloride.

During the concentration step, the pH of the liquid dairy stream will typically decrease slightly. Hence, the pH of the liquid dairy stream will after concentration typically be in the range of 5.0 to 5.9, such as 5.3 to 5.8. Preferably, the pH of the liquid dairy stream after the concentration step is in the range of 5.5 to 5.75.

The precipitation step:

In the method according to the invention, the concentrated liquid dairy stream is subjected to a precipitation step to precipitate calcium citrate, the precipitation step comprises either the precipitation in step A) or the precipitation in step B).

Step A) includes precipitation by heating the concentrated dairy stream to a temperature in the range of 40°C to 85°C for at least 5 minutes to precipitate calcium citrate. The higher the temperature, the shorter the period of time which is necessary for precipitation of calcium citrate. If the temperature is lower than 40°C, it will take a long time to precipitate calcium citrate. For example, if the temperature is 10°C, it will take 24 hours or longer to precipitate a small amount of calcium citrate.

The temperature for heating the concentrated dairy stream should preferably be in the range of 50°C to 80°C, and more preferably in the range of 55°C to 72°C, and even more preferably in the range of 60°C to 70°C.

Since the temperature and time are dependent of each other, the period of time should not be seen as a limitation. However, the period of time for performing the precipitation in step A) is typically in the range of 5 minutes to 2 hours, such as 10 minutes to 1.5 hour, preferably 15 to 60 minutes. If the temperature is in the range of 60°C to 70°C, the period for the precipitation is typically in the range of 15 to 60 minutes.

The precipitation may also be carried out by using precipitation step B). The precipitation step B) involves seeding of the concentrated liquid dairy stream and storing the seeded concentrated liquid dairy stream at a temperature of 10°C to 40°C.

The precipitation in step B), i.e. at lower temperatures increases the lag time of spontaneous nucleation and will not be feasible without seeding, since the precipitation then will take too long time. However, with the seeding and storage of the concentrated dairy stream at 10°C to 40°C, the precipitation of calcium citrate takes about 2 hours to 24 hours.

The term "nucleation" refers in the context of the present invention to what is normally understood with nucleation, namely formation of a thermodynamic new phase or a new structure. Nucleation is a mechanism which generates phase transitions and the start of a process of forming a new thermodynamic phase. In the present invention, seeding speeds up the phase transition of precipitating calcium citrate.

In an embodiment of the present invention, the seeding in step iii) B) is selected from the group consisting of direct seeding and indirect seeding. The direct seeding is by seeding with calcium citrate and indirect seeding is through a mechanical nucleation. Mechanical nucleation overcomes the activation energy of the nucleation, and mechanical nucleation may for example be by using ultrasound, kinetic energy, or heat energy. Direct seeding with calcium citrate is preferable.

Hence, in a preferred embodiment of the present invention, crystals of calcium citrate are added to the concentrated liquid dairy stream before the precipitation in step iii) B).

The precipitation step A) using precipitation at higher temperatures is preferred, since the precipitation of calcium citrate is obtained faster. Hence, the precipitation step A) is more feasible than precipitation step B).

In the precipitation steps, heating of the concentrated dairy stream may be obtained by using one or more devices selected from the group consisting of heat exchangers, steam infusion devices and direct steam injection devices.

The heat exchangers may be selected from tube heat exchangers and plate heat exchangers (PHE).

In a preferred embodiment, the heat treatment in step iii) A) is conducted as a two-step heat treatment, where the concentrated dairy product is first heated in a heat exchanger to a temperature of up to 50°C, and subsequently heated with direct steam injection or steam infusion to a temperature of up to 85°C. It is not desired to entirely use direct steam injection or steam infusion for heating, due to the steam consumption and dilution of the dairy liquid stream.

For heat treatment of a liquid dairy stream with a high solid content (degree of Brix), such as above 12°Bx, it is advantageous partly to use direct steam injection or steam infusion. Hence, it is desired to first use heat exchangers for heating to about 40-50°C followed by heating with direct steam injection or steam infusion to a higher temperature. Heating in heat exchangers to temperatures above 40-50°C will cause fouling in the heat exchangers if the solid content is too high.

This should be avoided. The higher the solid content, the lower the temperature which is required for rapid fouling in the heat exchanger. For example, a dairy liquid stream having a solid content of 10°Bx will not lead to fouling of the heat exchangers before the temperature is about 50°C. On the contrary, a liquid dairy stream concentrated to 16°Bx or higher will lead to fouling of the heat exchangers if heated to temperatures above 40°C.

However, in an embodiment of the present invention, the heat treatment in step iii) A) is conducted with direct steam injection or steam infusion to a temperature up to 85°C.

Optionally cooling:

In an embodiment of the invention, the concentrated and heat-treated liquid dairy stream may be cooled. The cooling in step iv) may for example be to a temperature in the range of 5°C to 15°C. The cooling step can be performed to decrease the risk of microbiological growth. The cooling is more preferably performed to a temperature in the range of 5°C to 10°C.

The cooling may be before or during the separation step v). For example, cooling may be in between two steps of separation using different devices for separation.

Separating precipitated calcium citrate:

In step v) of the method according to the present invention, the precipitated calcium citrate is separated from the concentrated liquid dairy stream by using one or more devices for mechanical separation of precipitated calcium citrate from the liquid dairy stream. In an embodiment of the invention, the one or more devices for separation in step v) is one or more selected from the group consisting of a clarifier, a centrifuge, a decanter, a filter, and a cyclone. The present invention should not be limited to the device of separation, since all types of devices known in the art that are suitable for separation could be used. The filter includes different types of filters, including membrane filtration, such as for example ultrafiltration.

In a preferred embodiment of the invention, the one or more devices for separation is a clarifier or ultrafiltration or a combination of a clarifier and ultrafiltration.

Two-step precipitation of calcium citrate and calcium phosphate:

In an embodiment of the present invention, the method involves a two-step precipitation and separation, first precipitate and separate calcium citrate and subsequently precipitate and separate calcium phosphate.

Hence, in an embodiment of the present invention, the method according the invention comprises that the liquid dairy stream obtained after separation of calcium citrate in step v) is subjected to a process of removing calcium phosphate, said process comprises the following steps:

a) concentrating the liquid dairy stream until the degrees of Brix is in the range of 15 to 30°Bx;
b) heating the concentrated liquid dairy stream to a temperature in the range of 40°C to 85°C;
c) adding an alkaline compound to the concentrated liquid dairy stream to adjust the pH to be at least 6.5 and holding for at least 5 minutes at 40°C to 85°C to precipitate calcium phosphate;
d) optionally cooling the heat-treated concentrated liquid dairy stream;
e) separating precipitated calcium phosphate from the heat-treated liquid dairy stream by using one or more devices for mechanical separation of precipitated calcium phosphate from the liquid dairy stream.

Concentration of the liquid dairy may be carried out by the processes earlier described for concentrating, i.e. one or more of reverse osmosis and nanofiltration. The concentration is preferably by use of reverse osmosis.

Heating may also be carried out by any of the devices for heating as earlier disclosed, such as one or more selected from the group consisting of heat exchangers, steam infusion devices, and direct steam injection devices.

The temperature and time of the heating step is similar to the temperature and time used for the precipitation of calcium citrate using heating (step A)) disclosed above.

An alkaline compound is added to the liquid dairy stream to increase the pH to be above 6.5. Preferably, the pH should be in the range of 6.5 to 8.0, and more preferably in the range of 6.8 to 7.8. Most preferably, the pH should be adjusted to be in the range of 7.0 to 7.5. The alkaline compound may typically be sodium hydroxide, but other alkaline compounds typically used for pH adjustment in food products may be used.

The conditions for the cooling step are also similar to the cooling step for the method of precipitation of calcium citrate, and may for example be cooling to a temperature in the range of 5°C to 15°C.

The one or more devices for separation is also similar to the one or more devices for separation earlier described for separating calcium citrate. Hence, the one or more devices for separation may be one or more selected from the group consisting of a clarifier, a centrifuge, a decanter, a filter, and a cyclone. The present invention should not be limited to the device of separation, since all types of devices known in the art that are suitable for separation could be used. The filter includes different types of filters, including membrane filtration, such as for example ultrafiltration.

In a preferred embodiment of the invention, the one or more devices for separation is a clarifier or ultrafiltration or a combination of a clarifier and ultrafiltration.

Preparation of lactose crystals:

The present invention also relates to a method of preparing crystallized lactose, wherein said method comprises the following steps:

A) providing the liquid dairy stream where calcium citrate has been removed according to the invention or the liquid dairy stream where calcium citrate and calcium phosphate have been removed according to the invention;
B) adjusting the pH to be in the range of 5.5 to 6.2
C) concentrating the pH adjusted liquid dairy stream of step B) until the degrees Brix is in the range of 55 to 65°Bx at a temperature of 50°C to 80°C;
D) cooling to a temperature of 5°C to 20°C to obtain crystallized lactose and mother liquor;
E) separating lactose crystals from the mother liquor;
F) optionally washing the lactose crystals with water.

In an embodiment, the concentration in step C) is by using an evaporator.

In a further embodiment, the separation of lactose crystals from mother liquor is by using a decanter.

The lactose crystals separated from the mother liquor may optionally be washed. In a preferred embodiment, the lactose crystals are washed with water.

The washed lactose crystals may then be separated from the washing water. This could for example be by using a centrifuge, ultrafiltration, or a clarifier.

The pH adjustment is typically obtained by adding a food grade acid. The acid used could be the same acids as earlier disclosed.

The pH adjusted liquid dairy stream obtained in step B) may in an embodiment of the invention be subjected to a decolouring step before being concentrated in step C). The decolouring step is any known decolouring step and may for example be by using active carbon.

The lactose crystals may in a further embodiment be dried to obtain a lactose powder. Drying is preferably obtained by using a fluid bed dryer.

The method of preparing crystallized lactose may also be obtained by using a liquid dairy stream where calcium phosphate has been removed as disclosed above but where calcium citrate has not been removed by the present method. Using a dairy liquid stream only subjected to removal of calcium phosphate may be relevant if a liquid dairy stream, that has not been subjected to addition of citric acid, is used.

It should be noted that embodiments and features described in the context of one of the aspects of the present invention also apply to the other aspects of the invention.

The invention will now be described in further details in the following non-limiting examples.

**Examples**

Example 1: Analysis of the calcium, citric acid and phosphor content in different ultrafiltration permeates to be used in lactose production

The purpose of this example is to show that an ultrafiltration permeate may have a different amount of calcium, citric acid, and phosphor. The amount of calcium, citric acid, and phosphor depends on the how the ultrafiltration permeate has been obtained.

The following methods were used:

- The content of calcium, potassium, magnesium, sodium, phosphor, and chloride was measured using the standard method AOAC 984.27, mod.
- The content of chloride was measured using potentiometric titration method, ISO 5943/IDF 88, fourth edition, 2006.
- The ash content was measured using the method NMKL 173 2nd edition, 2005.
- The total protein content was measured using the Kjeldahl method, ISO 8968-3:2004(E)/IDF 20-3:2004: Determination of nitrogen content, Block-digestion method (semi-micro rapid routine method) ISO 8968-3:2007/IDF 20-3:2007.
- Non-protein-nitrogen was measured using ISO 8968-2001/IDF 20-4:2001: Determination of nitrogen content. Determination of non-protein-nitrogen content.

- The total solid content was measured using the method: ISO 6731:2010E/IDF21:2020E modified "Milk, cream and evaporated milk. Determination of the total solids content".
- The content of citric acid is determined by the method of Boehringer Mannheim, test-kit, Cat no 10 139 076 035.
- The lactose and galactose content is measured using the method: DS/ISO 5765-2:2002, IDF 79-2, First edition 2002-09-01, "Dried milk, Dried ice-Mixes & Processed Cheese - Determination of lactose content, enzymatic methods, Boehringer Mannheim test-kit, Cat. No. 176 303. Modified - Carrez I and II, NaOH

In table 1 below, the content of calcium, phosphor, citric acid, and lactose is shown for two different ultrafiltration permeates. The first ultrafiltration permeate (10 samples used), referred to as "UF permeate high calcium", is obtained in connection with the preparation of cheese whey where citric acid has been added to the skim milk before ultrafiltration. The "UF permeate high calcium" is a ultrafiltration permeate obtained from ultrafiltration of skim milk that has been pH-adjusted with citric acid added before concentration.

The second ultrafiltration permeate (5 samples used), referred to as "UF permeate low calcium" is obtained in connection with the UF-concentration of sweet whey. For these samples, no pH-adjustment with citric acid has been applied to the whey before ultrafiltration. The "UF permeate low calcium" is obtained from cheese milk that has been subjected to renneting, separation of curds from whey and ultrafiltration of said obtained whey.

The samples of UF permeates are concentrated to 25% solid content by use of reverse osmosis before analysis.

Table 1: Percent by weight of total solid content of various nutrients in UF permeates

|  |  | UF permeate high calcium | | UF permeate low calcium | |
|---|---|---|---|---|---|
|  |  | Average | Stdev*** | Average | Stdev*** |
| Ash | % of TS* | 10.752 | 0.710 | 9.520 | 0.452 |
| Calcium | % of TS | 1.369 | 0.088 | 0.730 | 0.019 |
| Chloride | % of TS | 1.796 | 0.074 | 1.854 | 0.051 |
| Potassium | % of TS | 2.639 | 0.066 | 2.563 | 0.045 |
| Magnesium | % of TS | 0.158 | 0.010 | 0.141 | 0.000 |
| Sodium | % of TS | 0.655 | 0.021 | 0.650 | 0.022 |
| NPN** | % of TS | 0.477 | 0.085 | 0.413 | 0.040 |
| Phosphor | % of TS | 1.096 | 0.039 | 0.855 | 0.009 |
| Total protein | % of TS | 3.385 | 0.083 | 2.673 | 0.023 |
| Citric acid | % of TS | 5.385 | 0.226 | 2.959 | 0.031 |
| Galactose | % of TS | 0.372 | 0.021 | 1.034 | 0.040 |
| Lactose | % of TS | 81.514 | 2.619 | 81.286 | 1.231 |
| pH |  | 5.967 |  | 5.600 |  |
| * TS refers to total solid content<br>** NPN refers to non-protein nitrogen<br>*** Stdev refers to standard deviation | | | | | |

Hence, table 1 shows that the calcium content is higher in an ultrafiltration permeate obtained after addition of citric acid to skim milk (calcium content is 1.369% by weight of TS) as compared to the calcium content in an ultrafiltration permeate of whey (citric acid has not been added to the whey before ultrafiltration of it; calcium content is 0.730% by weight of TS).

In addition, table 1 shows that the phosphor content is higher in an ultrafiltration permeate obtained after addition of citric acid to skim milk (phosphor content is 1.096% by weight of TS) as compared to the calcium content in an ultrafiltration permeate of whey (phosphor content is 0.855% by weight of TS).

Furthermore, the content of citric acid is higher in the ultrafiltration permeate obtained after the addition of citric acid to skim milk as compared to the citric acid content in an ultrafiltration permeate of whey (5.385% versus 2.959%).

There will be some citric acid present in the UF permeate of whey, because milk (from which whey is derived) will comprise a low amount of citric acid.

The lactose content is similar in the two ultrafiltration permeates.

In table 2 below, the comparison between the total solid composition for the UF permeate high calcium" and the "UF permeate low calcium" is shown. The "UF permeate low calcium" nutrients are indexed as 100.

Table 2:

|  | UF-permeate low calcium | UF-permeate high calcium |
|---|---|---|
|  | Index | Index |
| Ash | 100 | 113 |
| Calcium | 100 | 188 |
| Chloride | 100 | 97 |
| Potassium | 100 | 103 |
| Magnesium | 100 | 112 |
| Sodium | 100 | 101 |
| Phosphor | 100 | 128 |
| Citric acid | 100 | 182 |
| Lactose | 100 | 100 |

From table 2, it is shown that the "UF permeate high calcium" in comparison to the "UF permeate low calcium" typically contains:

- about 80% higher content of calcium
- about 30% higher content of phosphor
- about 80% higher content of citric acid

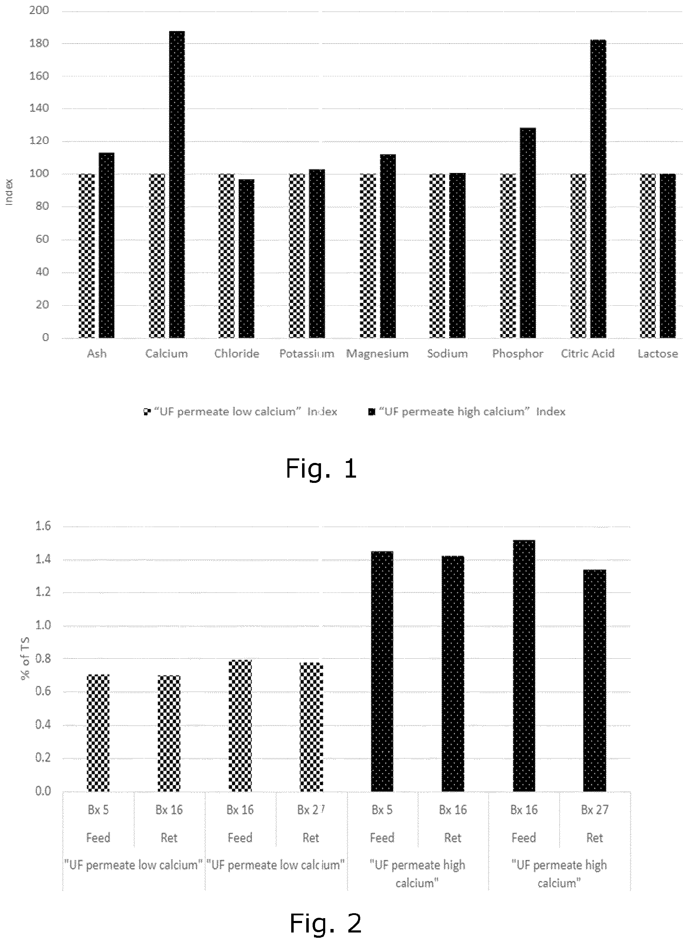

The index numbers shown in table 2 are also shown in figure 1.

In conclusion, different fractions of ultrafiltration permeates obtained in connection with preparing cheese may have different amounts of calcium, citric acid, and phosphor.

Example 2: Effect of feed type on performance of concentration by membrane filtration

The purpose of this example is to show the difference in the performance of reverse osmosis of a "UF permeate high calcium" and a "UF permeate low calcium". The "UF permeate high calcium" and "UF permeate low calcium" are the same ultrafiltration permeates as used in example 1.

The ultrafiltration permeates were collected and two trials of reverse osmosis (RO) membrane filtration were conducted to concentrate the UF permeates to:

- RO concentration from 5°Bx to 16°Bx (26 Bar transmembrane pressure)
- RO concentration from 16°Bx to 27°Bx (46 Bar transmembrane pressure)

The pressure drop across the membrane was kept at 0.8 Bar. Each step was performed with a single loop in continuous mode. The filtration plant was equipped with 2 tubes each containing two 4" RO elements from Toray.

The calcium, phosphor and citric acid content (% by weight of total solid content) was measured of the below mentioned samples using the methods disclosed in example 1:

- UF permeate high calcium as feed, 5°Bx
- UF permeate low calcium as feed, 5 °Bx
- RO retentate of UF permeate high calcium, Brix 5, as feed concentrated by RO to Brix 16
- RO retentate of UF permeate low calcium, Brix 5, as feed concentrated by RO to Brix 16
- UF permeate high calcium as feed, Brix 16
- UF permeate low calcium as feed, Brix 16
- RO retentate of UF permeate high calcium as feed, Brix 16, concentrated by RO to Brix 27
- RO retentate of UF permeate low calcium as feed, Brix 16, concentrated by RO to Brix 27

Figure 2 shows the calcium content in percentage of the total solid content in the different samples, and figure 2 shows that the samples with "UF permeate high calcium" contain much more calcium as compared to the samples with "UF permeate low calcium".

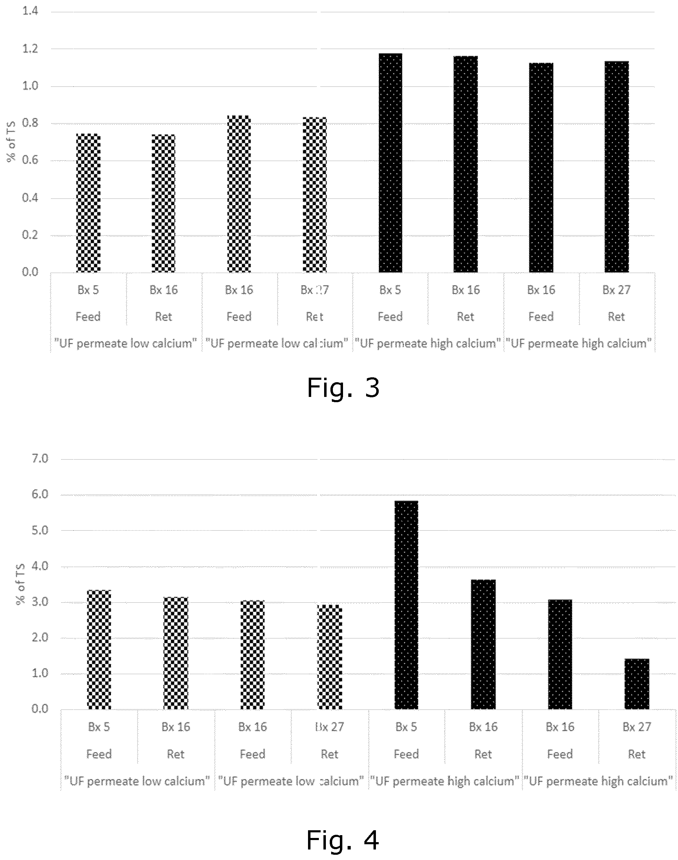

Figure 3 shows the content of phosphor in percentage of the total solid content in the different samples. Figure 3 shows that the samples with "UF permeate high calcium" contain much more phosphor as compared to the samples with "UF permeate low calcium".

Figure 4 shows the content of citric acid in percentage of total solid content in the different samples. As expected, the content of citric acid is higher in the samples "UF permeate high calcium" than in the samples with "UF permeate low calcium". Figure 4 also shows that the content of citric acid in the feed and RO retentate is varying a lot for the samples with "UF permeate high calcium". The variation in citric acid is believed to due to precipitation of calcium citrate during storage. The different samples were taken during pilot trials and kept at cold conditions until analysed. However, due to logistics, the analysis of the samples could be performed after different residence time of the samples. It was observed that after storage, some of the samples comprised precipitated material. To analyse this further, some samples of feed and RO retentate were stored at room temperature and it was observed that the samples went turbid with time. The increased turbidity over time is an indication of chemical precipitations. The inventors of the present invention believe that the precipitations are calcium citrate precipitations.

Example 3: Analysis of fouling of reverse osmosis (RO) membrane

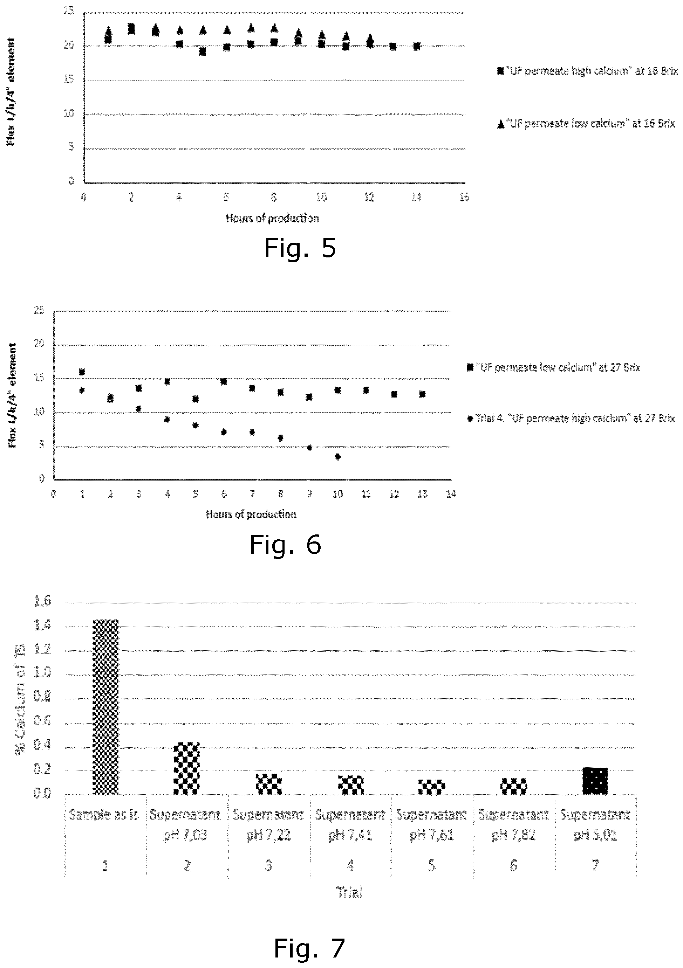

The flux data for concentration over a RO membrane were analysed with respect to possible fouling of the membrane. Flux data were analysed for continuously RO concentration from 5°Bx to 16°Bx for samples of "UF permeate high calcium" and "UF permeate low calcium". The RO concentration was performed continuously at 26 bar TMP (transmembrane pressure) for 4.5 hours. These flux data are shown in figure 5.

In addition, flux data were analysed for continuously RO concentration from 16°Bx to 27°Bx for the samples of "UF permeate high calcium" and "UF permeate low calcium". The RO concentration was performed continuously at 46 bar TMP for 4.5 hours. These flux data are shown in figure 6.

The flux data are measured as L/h/element where "element" refers to "spiral wound membrane element".

Figure 5 shows that the flux data for "UF permeate high calcium" and "UF permeate low calcium" are very similar when concentrating over the RO membrane from 5°Bx to 16°Bx. The measurements for both feeds result in fluxes at about 20-22 L/h/element, and there are therefore no significant signs of fouling within the 4.5 hours trial.

Figure 6 shows the flux of both the "UF permeate high calcium" and "UF permeate low calcium" when concentrating from 16°Bx to 27°Bx. The flux is about 14-15 L/h/element for both UF permeates at the beginning (16°Bx), but the flux of the "UF permeate high calcium" decreases a lot over time which is an indication of severe fouling of the membrane. After 4.5 hours, the flux of RO concentrating the "UF permeate high calcium" is decreased to 3.5 L/h/element or just 25% of the initial flux. On the contrary, the flux of concentrating the "UF permeate low calcium" from 16°Bx to 27°Bx is not decreased significantly and hence shows no signs of fouling.

Hence, this example demonstrates that the "UF permeate high calcium" is much more prone to fouling of the RO membrane during RO concentration at high concentration (high solid content) compared to "UF permeate low calcium". Hence, the "UF permeate high calcium" cannot be used directly in a process for preparing lactose if the feed is concentrated to a Brix of 27°Bx without substantial reduction in the capacity.

Furthermore, the RO process will be very expensive with a feed similar to "UF permeate high calcium", since the RO plant will foul, cleaning will be needed more often, and more frequent change of the membrane will be needed, and the production costs will therefore be very high.

Example 4:

The purpose of this example is to show how precipitation of calcium citrate and calcium phosphate can be made by controlling the pH.

The methods disclosed in example 1 were used to analyse the samples.

The "UF permeate low calcium" and "UF permeate high calcium" were collected and frozen for about one week before being thawed and used for analysis.

The method showing effect/control of the pH on precipitation of calcium citrate and calcium phosphate involved the following process:

Step 1: Samples of "UF permeate high calcium" and "UF permeate low calcium" were heated in a water bath to 80°C
Step 2: Sodium hydroxide, 27% (w/w), was added to adjust the pH to various levels
Step 3: The pH adjusted samples were held for 60 min at 80°C
Step 4: The samples were centrifuged at 3000 G for 5 minutes
Step 5: The supernatants obtained after centrifugation were decanted and cooled to 8-10°C

The supernatants obtained were subjected to analysis. Further, the pH was measured for the samples after heating in step 1 and after cooling of the supernatant (the pH was adjusted for the samples where NaOH was added).

In table 3 below, the pH and the content of phosphor, calcium and citric acid is shown for samples of "UF permeate high calcium" having different pH values.

Table 3:

| Sample No. | Sample | Heating 80°C for 60 min | pH adj. with NaOH | pH** | pH*** | Phosphor % (w/w) | Calcium % (w/w) | Citric acid % (w/w) | Phosphor % of TS | Calcium % of TS | Citric acid % of TS |
|---|---|---|---|---|---|---|---|---|---|---|---|
| UF permeate High calcium | | | | | | | | | | | |
| 1 | Sample as is* | No | No | - | 5.52 | 0.298 | 0.307 | 1.300 | 1.42 | 1.46 | 6.19 |
| 2 | Supernatant pH 7.03 | Yes | Yes | 7.03 | 6.61 | 0.175 | 0.091 | 1.130 | 0.85 | 0.44 | 5.47 |
| 3 | Supernatant pH 7.22 | Yes | Yes | 7.22 | 6.85 | 0.140 | 0.036 | 1.120 | 0.69 | 0.18 | 5.54 |
| 4 | Supernatant pH 7.41 | Yes | Yes | 7.41 | 6.99 | 0.144 | 0.034 | 1.130 | 0.70 | 0.17 | 5.48 |
| 5 | Supernatant pH 7.61 | Yes | Yes | 7.61 | 7.32 | 0.136 | 0.025 | 1.140 | 0.67 | 0.12 | 5.58 |
| 6 | Supernatant pH 7.82 | Yes | Yes | 7.82 | 7.40 | 0.124 | 0.029 | 1.170 | 0.60 | 0.14 | 5.65 |
| 7 | Supernatant pH 5.01 | Yes | Yes | 5.01 | 4.85 | 0.253 | 0.049 | 0.670 | 1.20 | 0.23 | 3.18 |
| UF permeate Low calcium | | | | | | | | | | | |
| 8 | Sample as is* | No | No | - | 5.63 | 0.217 | 0.203 | 0.76 | 0.87 | 0.81 | 3.05 |
| 9 | Supernatant pH 7.04 | Yes | Yes | 7.04 | 6.65 | 0.116 | 0.028 | 0.75 | 0.45 | 0.11 | 2.92 |
| 10 | Supernatant pH 7.22 | Yes | Yes | 7.22 | 6.84 | 0.109 | 0.019 | 0.76 | 0.41 | 0.07 | 2.87 |
| 11 | Supernatant pH 7.36 | Yes | Yes | 7.36 | 7.05 | 0.107 | 0.018 | 0.73 | 0.42 | 0.07 | 2.88 |
| 12 | Supernatant pH 7.44 | Yes | Yes | 7.44 | 2.11 | 0.106 | 0.017 | 0.74 | 0.41 | 0.06 | 2.83 |
| *The "sample as is" is the "UF permeate high calcium" or "UF permeate low calcium" **pH after heating measured at 80°C - before centrifugation ***pH in supernatant - measured at 10°C | | | | | | | | | | | |

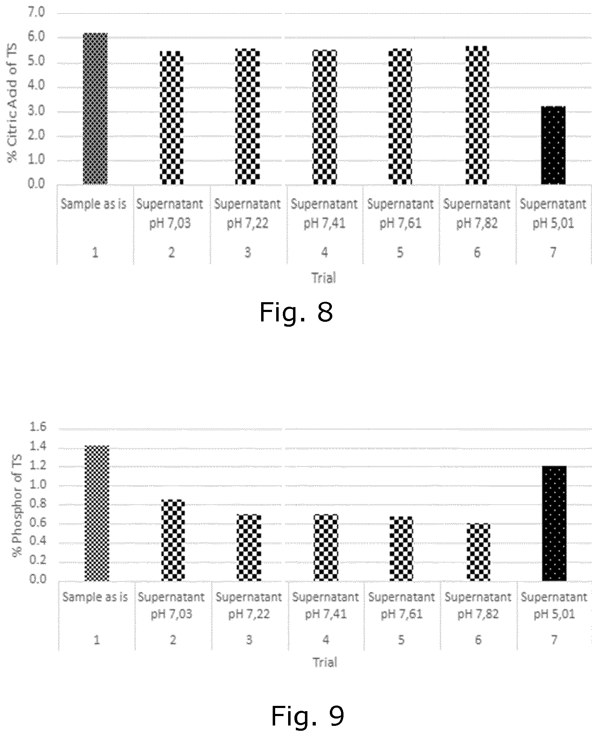

Figure 7 shows the calcium content of the total solid content in the different samples, while figure 8 shows the citric acid content of the total solid content in the different samples. Figure 9 shows the phosphor content of the total solid content in the different samples.

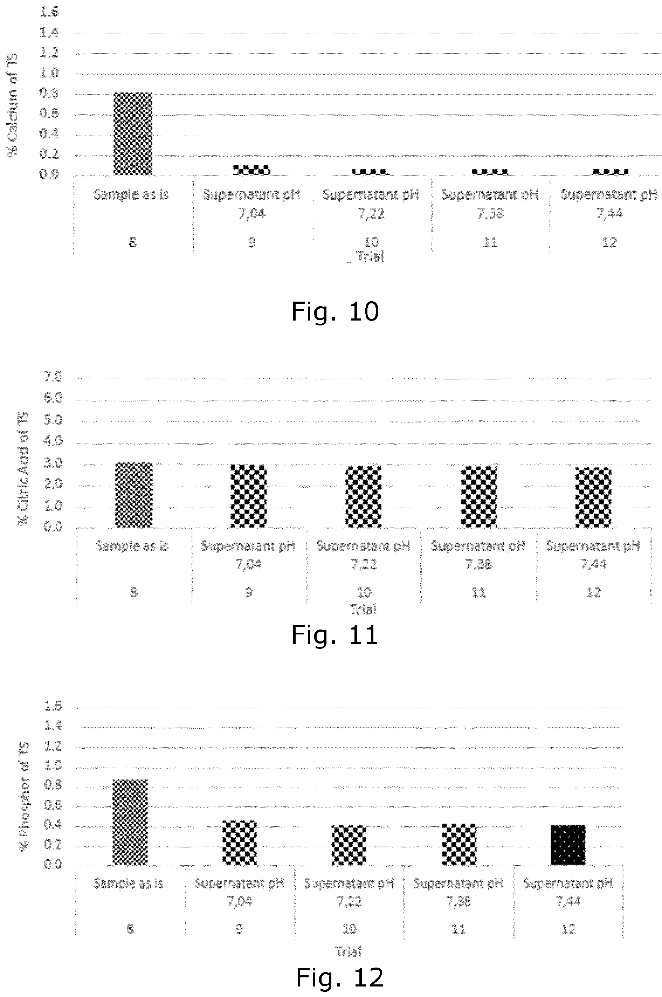

Table 3 and figure 7 show that for the "UF permeate high calcium", the calcium content is reduced significantly in samples 2-7 as compared to the sample of "UF-permeate high calcium" as it is. The same can be seen in table 3 and figure 10 for the samples with "UF permeate low calcium".

Table 3 and figure 8 show that for the "UF permeate high calcium", the citric acid content is slightly reduced in samples 2-6 as compared to the sample of "UF permeate high calcium" as it is. In sample 7, sodium hydroxide was not added and the citric acid level is therefore reduced significantly more than in the trials 2-6 where sodium hydroxide was added. In table 3 and in figure 11, showing samples of "UF permeate low calcium", it is shown that the citric acid content is almost the same in all samples. Hence, it can be concluded that citric acid is poorly removed as calcium citrates at pH around 7-8, but is removed at lower pH around 5.

Table 3 and figure 9 show that for the "UF permeate high calcium", the phosphor content is reduced in samples 2-6 as compared to the sample of "UF permeate high calcium" as it is. In sample 7 where no sodium hydroxide was added, the phosphor is not reduced to the same extent as in the samples 2-6 where sodium hydroxide was added. The same reduction of phosphor is observed for the samples with "UF permeate low calcium", see table 3 and figure 12. Hence, it can be concluded that phosphor is removed as calcium phosphate at pH around 7-8, but is poorly removed at lower pH such as around 5.

Hence, this example shows that removal of calcium, citrate and phosphate can be controlled by the pH and that calcium citrates are precipitated at a low pH while calcium phosphates are precipitated at a high pH.

Example 5: Effect of pH on precipitation product and the use of precipitation in 2 steps to reduce fouling

The purpose of this example is to show how the pH can be used to control the precipitation product and to develop a process that would reduce the evaporator fouling potential of the "UF permeate high calcium" to the same level as the "UF-permeate low calcium" such that the "UF permeate high calcium" is suitable to be used for production of lactose.

The analytical methods described in example 1 were used to analyse the samples. The "UF permeate low calcium" and "UF permeate high calcium" were collected and frozen for about one week before being thawed and used for analysis. The analysis was divided into two parts:

- Part 1: 1 step precipitation
- Part 2: 2 step precipitations

Part 1: 1 step precipitation

The precipitation with only one precipitation step involved a screening of the pH from 4.8 to 7.8 using both "UF permeate low calcium" and UF permeate high calcium".

The method of making the one precipitation step comprised:

a) heating the sample in a water bath to 80°C
b) adding NaOH, 27% (w/w), to the heated sample to adjust pH if needed
c) holding the sample for 60 minutes at 80°C
d) centrifugation of the sample from step c) at 3000G for 5 minutes
e) decanting the supernatant and cooling the supernatant to 8-10°C

Part 2: 2 step precipitation

The first step of precipitation was:

i) the sample was heated in a water bath to 5°C, 15°C or 80°C
ii) holding the sample for 60 minutes at the specified temperature
iii) centrifugation of the sample from step ii) at 3000G for 5 minutes
iv) decanting the supernatant, and the supernatant is ready for the second precipitation step

The second step of precipitation was:

v) The sample from step iv) was heated in water bath to 80°C
vi) adding NaOH, 27% (w/w), or $Ca(OH)_2$ to the heated sample to adjust pH, if needed
vii) holding the sample for 60 minutes at 80°C

viii) centrifugation of the sample from step vii) at 3000G for 5 minutes

ix) decanting the supernatant and cooling the supernatant to 8-10°C

Five different combinations of 1st step precipitation and 2nd step precipitation were made in part 2. This is shown in table 4 below.

Table 4:

| Number | 1st precipitation step | | 2nd precipitation step | |
|---|---|---|---|---|
| | Temperature 1 | pH1 | Temperature 2 | pH2 |
| 1 | 5°C | As is* | 80°C | NaOH, pH 7 |
| 2 | 80°C | As is* | 80°C | NaOH, pH 7 |
| 3 | 80°C | As is* | 80°C | Ca(OH)$_2$, pH 7 |
| 4 | 5°C | As is* | 80°C | As is |
| 5 | 15°C | As is* | 80°C | NaOH, pH 7 |
| * The "as is" pH was measured to 5.3 | | | | |

Ca(OH)$_2$ was included in the analysis to investigate whether the additional calcium for precipitation of citrate would be beneficial, and if the level of phosphor could be decreased further. The NaOH and Ca(OH)$_2$ addition was performed to a target pH 7 measured at 80°C before the precipitation.

In the process of preparing lactose, the main concern regarding the processability of the "UF permeate high calcium" is in the evaporator. Experience has shown that evaporator fouling typically consists of calcium citrate.

Hence, to evaluate the fouling potential of a sample, the solubility product was calculated:

$$K = [Ca]^3 * [citrate]^2$$

The density was not measured on the specific samples. Hence, the unit used is mol/kg.

Results

Part 1 - only 1 step precipitation

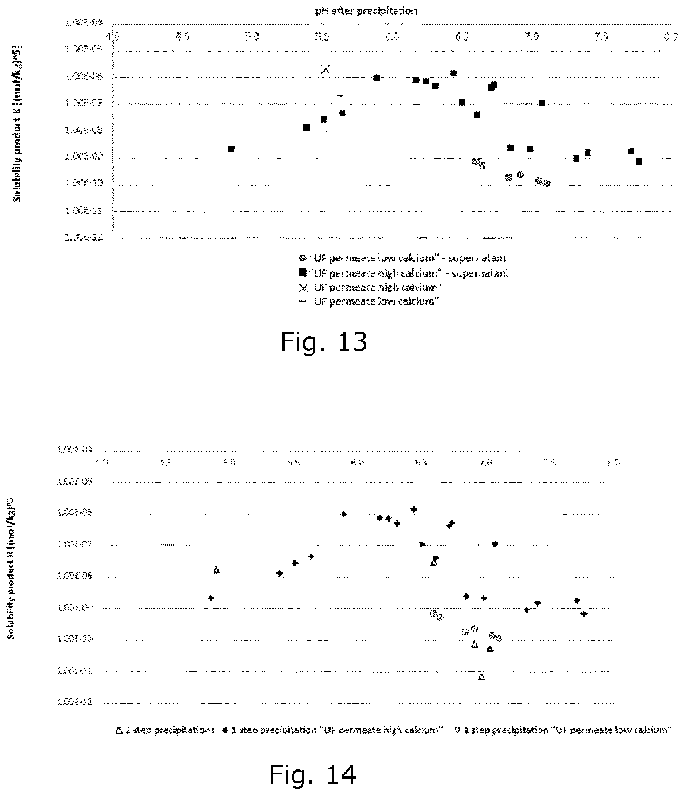

In figure 13, the calculated solubility product is plotted against the pH measured in the cooled sample after separation of the precipitate. It is shown for samples of "UF permeate high calcium" and "UF permeate low calcium", respectively.

It is shown in figure 13 that the solubility product of "UF permeate high calcium" (squares) is higher than the solubility product of "UF permeate low calcium" (circles). Further, figure 13 shows that the highest solubility product for "UF permeate high calcium" is found around pH 6, where the precipitate mainly consists of calcium phosphate and a lot of the citrate is maintained in the supernatant, while the calcium concentration is not as low as seen at higher pH values. At pH values around 7-8, the solubility product is lower because very little calcium is maintained in the supernatant for citrate to bound with and precipitate as calcium citrate. At a pH of 5.5 and lower, citrate is precipitated together with calcium which affects the solubility product.

Hence, it can be concluded that if a precipitation is made at pH around 6.5 and higher, the fouling potential is high if "UF permeate high calcium" is used.

Part 2 - both 1 step precipitation and 2 step precipitation

In figure 14, the calculated solubility product is plotted against the pH measured in the cooled samples after separation of the precipitate.

It is shown for:

- 2-step precipitation (triangles)
- 1 step precipitation "UF permeate high calcium"
- 1 step precipitation "UF permeate low calcium"

Figure 14 shows that in the 2 step precipitation, a low solubility product was obtained where calcium citrate is precipitated first at 80°C with a feed that has not been pH adjusted (pH about 5.3) before calcium phosphate is precipitated by adjusting the pH to 7 with NaOH.

In addition, the use of $Ca(OH)_2$ instead of NaOH for the pH adjustment was analysed. In this analysis, precipitation at low pH values was performed at 15°C instead of 80°C. Using $Ca(OH)_2$ for the pH adjustment also showed an acceptable solubility product, but the solubility product was slightly higher than using precipitation at 80°C and pH adjustment with NaOH.

In conclusion, example 5 shows that the precipitation of calcium citrate and calcium phosphate can be controlled by adjusting the pH of the feed. Further, example 5 shows that the use of a two-step precipitation of first calcium citrate at a low pH and subsequently calcium phosphate at a higher pH results in a lower fouling potential.

Example 6: Precipitation using low temperature and long residence time

The purpose of this example is to show how calcium citrate can be precipitated at low temperature and long residence time.

The feed used for this example is a "UF permeate high calcium" obtained by collecting a sample of an ultrafiltration permeate of a milk with citric acid added. The "UF permeate high calcium" feed was concentrated by reverse osmosis (RO) to Brix 20.

The feed was transferred to reactors where the temperature was set to 15°C. The analysis lasted 21 hours and samples were collected for analysis after 0, 4, 8, and 18 hours storage. The samples were immediately separated by centrifugation at 3000g for 5 minutes, diluted 1:1 with RO polished water, cooled to 5°C and sent for analysis.

Two reactors were used, one reactor only comprised the feed, but in the second reactor 0.75g of calcium citrate precipitate was added to 2L of feed as seeding. Interpolations between the data points were made by fitting a Weibull distribution, but are only meant as a guide for the eyes. A slight extrapolation was included to estimate the residence time needed without seeding.

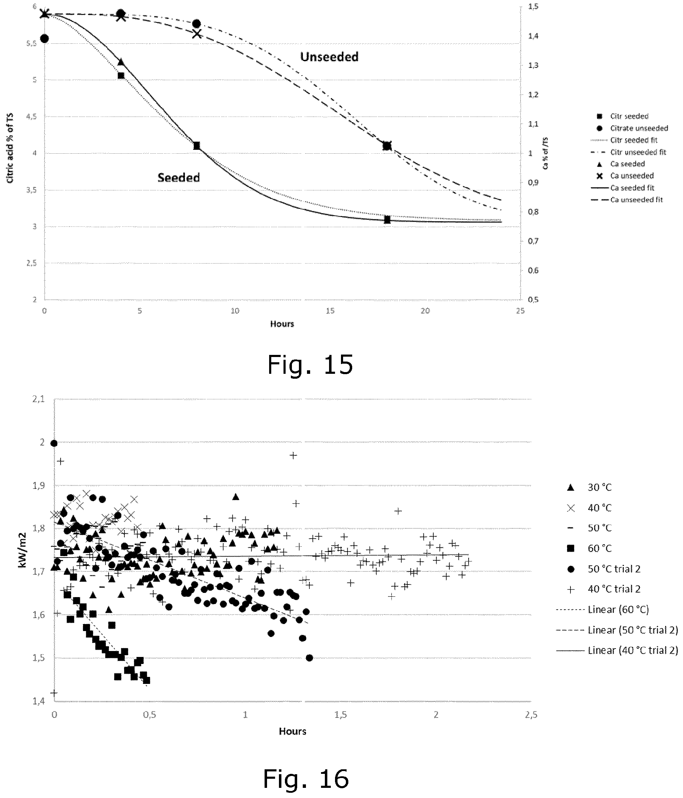

The result is shown in figure 15. Figure 15 shows the development in calcium and citric acid concentration per total solids in the supernatant fractions over the period of time of the analysis. The two vertical axes are scaled to show how the decline in calcium (right axis) and citric acid (left axis) concentrations are correlated.

For the seeded trial, the calcium (triangles) and citric acid (squares) concentrations have decreased considerably already after 4 hours. The rate of change is lower between the last 2 samplings at 8 and 18 hours suggesting that the concentrations are approaching equilibrium.

For the unseeded samples, the calcium concentration (crosses) only begins to decrease after 8 hours and the citrate concentration (circles) measurements are actually higher after both 4 and 8 hours than the feed. The most likely explanation is that the feed sample has precipitated before the analysis which makes part of the citric acid unavailable for the enzymatic analysis. After 18 hours, the unseeded trial had only precipitated about a third of the calcium and citric acid whereas the seeded trial had precipitated about half of the calcium and citric acid. The Weibull fit of the calcium and citric acid measurements from the unseeded trial suggests that it would require 1 day or longer to approach the equilibrium concentration.

In conclusion, this example demonstrates that at 15°C the precipitation of calcium citrate requires 1 day or longer of residence time to approach equilibrium at 20°Bx, but that the required residence time can be reduced to 18 hours or shorter by seeding the feed solution with previously precipitated calcium citrate.

If a sample is used having a Brix higher than 20°Bx, the precipitation time will be decreased, but the filtration plant will more likely foul due to the higher calcium level. Therefore, it is preferred to carry out the precipitation and removal of the precipitated material before further concentration in a filtration plant if having a feed with a high calcium and citric acid content, i.e. a feed similar to "UF permeate high calcium".

Example 7: Precipitation using high temperature and short residence time

The purpose of this example is to show how calcium citrate can be precipitated at high temperature and short time.

The feed used is a "UF permeate high calcium" obtained by collecting a sample of an ultrafiltration permeate of a milk with citric acid added. The "UF permeate high calcium" feed was concentrated by reverse osmosis (RO) to Brix 16.

The concentrated feed was heated to 40°C on plate heat exchangers (PHE) in continuous mode. The heated concentrated feed was collected in a 1200 L tank and stored for to hours. The product in the tank was then further heated to 60°C using direct steam injection for about 12 minutes. Samples were collected at different time points during the precipitation. The samples were separated by centrifugation at 3000g for 5 minutes and cooled to 5°C. The citric acid content in the supernatant was measured using a Foss Milkoscan FT1.

The plate heat exchanger used for all examples is from FH Scandibox A/S, serial No. 115144.

In table 5 below, the content of citric acid measured in the different samples collected is shown.

Table 5:

| Time [min] | Citric acid (%) | Citric acid of TS** (%) | Temperature |
|---|---|---|---|
| Feed | 0.81 | 5.5 | 8 |
| PHE* exit | 0.77 | 5.3 | 40 |
| -6 | 0.73 | 5.1 | 50 |
| 0 | 0.45 | 3.2 | 60 |
| 10 | 0.31 | 2.2 | 60 |
| 20 | 0.29 | 2.1 | 60 |
| 30 | 0.28 | 2.0 | 60 |
| 60 | 0.26 | 1.0 | 60 |
| *PHE refers to plate heat exchangers<br>**TS refers to total solid content | | | |

Table 5 shows that the precipitation of citric acid is very slow at 50°C, where the citric acid concentration has only decreased by 0.08 g/100 g during the 2 hours it takes to fill the tank at 40°C and heat it to 50°C. However, during the next 6 minutes of heating from 50°C to 60°C, the concentration of citric acid has decreased by 0.28g/100g. After 10 minutes of heating at 60°C, the concentration of citric acid has further decreased and after 60 minutes of heating at 60°C, the concentration of citric acid is only about 32% of that originally in the feed. Further, it is observed that after 10 minutes of heating at 60°C, about 90% of the yield of citric acid is obtained and after 10 minutes at 60°C, the precipitation is again slow.

In conclusion, this example shows that at 60°C a residence time of 10-60 minutes is enough to achieve a high yield of removal of citric acid.

Example 8: Analysis of heating of "UF permeate high calcium"

The purpose of this example is to show the constraints of heating on a product with high fouling potential.

The feed used is a "UF permeate high calcium" obtained by collecting a sample of an ultrafiltration permeate of a milk with citric acid added. The "UF permeate high calcium" feed was concentrated by reverse osmosis (RO) to Brix 16. The concentrated feed was heated to various temperatures on a plate heat exchanger (PHE) in continuous mode and samples were taken.

The concentrated feed was heated to 30°C, 40°C, 50°C, and 60°C in short runs of about 30 minutes. The change from 50°C to 60°C was done without immediate cleaning.

The heat transfer coefficient was calculated at different residence times for the samples heated to different temperatures. The heat flow, Q, was calculated from the service water flow, $Q_w$, heat capacity, $C_p$, and temperature change, dT:

$$Q = Q_w * C_p * dT$$

The heat transfer coefficient, U, is calculated from low mean temperature, LMDT, and plate area, A.

$$Q = Q_w * LMTD * A,$$

where $LMDT = (dT_1 - dT_2)/(\ln(dT_1)-\ln(dT_2))$ and $dT_1$ and $dT_2$ are temperature differences between the streams in each end of the plate heat exchanger. Further, the pressure was measured.

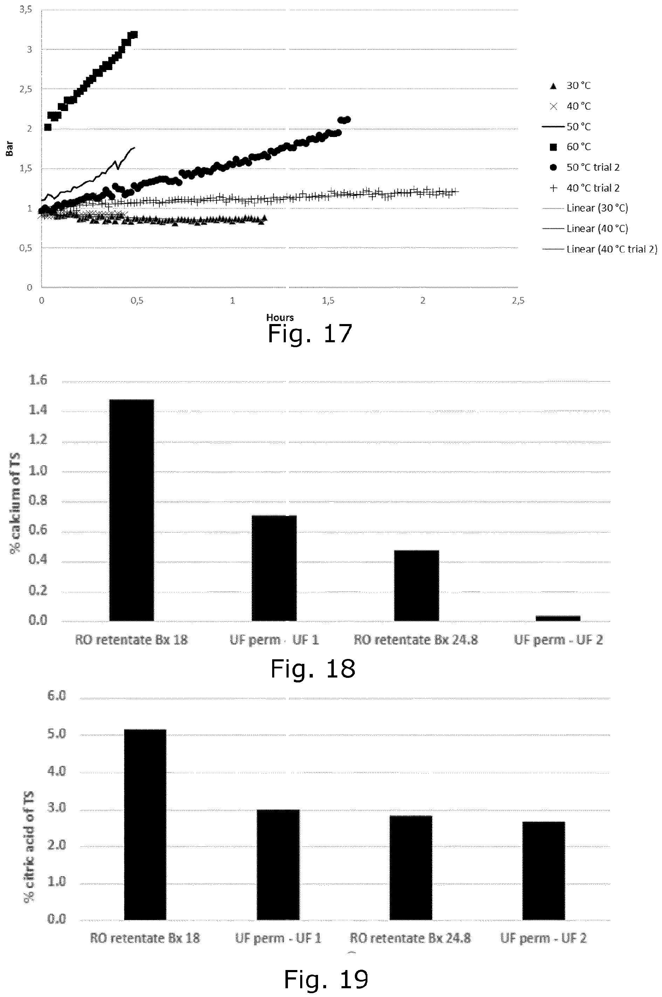

Figure 16 shows the heat transfer coefficient measured of the samples in the analysis while the pressure drop over the PHE can be seen in figure 17.

Figures 16 and 17 show that heating to 30°C (triangles) and 40°C (crosses) using plate heat exchangers did not result in any changes in heat transfer coefficient or pressure drop. This is an indication that those temperatures did not result in fouling of the plate heat exchanger.

At 50°C (circles), the pressure drop is increasing, but the heat transfer coefficient is not varying much during the 30 minutes analysis time. At 60°C, the heat transfer coefficient immediately started to decline and the increase in pressure drop accelerated.

17

In conclusion, this example shows that heating of the "UF permeate high calcium" concentrated to 16°Bx on a plate heat exchanger is stable with only small amounts of fouling up to 40°C. However, heating to 50°C in the plate heat exchanger would result in fouling and would at least require an increased frequency of cleaning. Heating in the plate heat exchanger to 60°C results in rapid fouling.

Example 9: 2-step precipitation of calcium citrate and calcium phosphate

The purpose of this example is to show that calcium citrate can be precipitated from a "UF permeate high calcium" in a first step (step 1) at a low pH and calcium phosphate precipitated in a second step (step 2) at a higher pH.

The feed used is a "UF permeate high calcium" obtained by collecting a sample of an ultrafiltration permeate of a milk with citric acid added. The "UF permeate high calcium" feed was concentrated by reverse osmosis (RO) to 18°Bx.

The precipitation method was carried out as mentioned below:

Step 1 precipitation at low temperature and low pH:

- The "UF permeate high calcium" concentrated to 18°Bx was heated on a plate heat exchanger to 25°C and stored in a tank at 25°C for 16 hours
- After 16 hours storage, calcium precipitates was observed in the tank.
- The stored "UF permeate high calcium" was ultrafiltrated (UF1) using the Alfa Laval GR82PE ultrafiltration membrane.

Step 2 precipitation at high temperature and high pH:

- The UF permeate from UF1 was concentrated by reverse osmosis to Brix 24.8.
- The RO retentate having a pH of 4.88 at 7°C was stored in a tank for about 89 hours.
- The concentrated RO retentate having a Brix of 25.8 was heated with direct steam in the tank to 80°C and the pH was adjusted to 6.5 with 7% (w/w) NaOH after heating. The Brix is then 21.1 due to addition of NaOH and direct steam.
- The pH adjusted RO retentate was kept at 20°C.
- The pH adjusted and heated RO retentate was ultrafiltrated using the same UF membrane as in step 1, and cooled to 3-5°C.

The content of calcium, phosphor, citric acid, ash, protein and the pH were measured using the methods disclosed in example 1. The data are shown in table 6 below based on the total solid content.

Table 6:

| | RO retentate Brix 18 | UF permeate - UF1 | RO retentate brix 24.8 | UF permeate - UF2 |
|---|---|---|---|---|
| Ash (% of TS) | 10.61 | 9.34 | 9.05 | 8.53 |
| Calcium (% of TS) | 1.48 | 0.71 | 0.48 | 0.04 |
| Phosphor (% of TS) | 1.15 | 1.18 | 1.09 | 0.80 |
| Protein (% of TS) | 2.91 | 2.93 | 2.66 | 2.77 |
| Citric acid (% of TS) | 5.15 | 2.99 | 2.83 | 2.67 |
| pH | 5.470 | 5.480 | 4.890 | 7.190 |

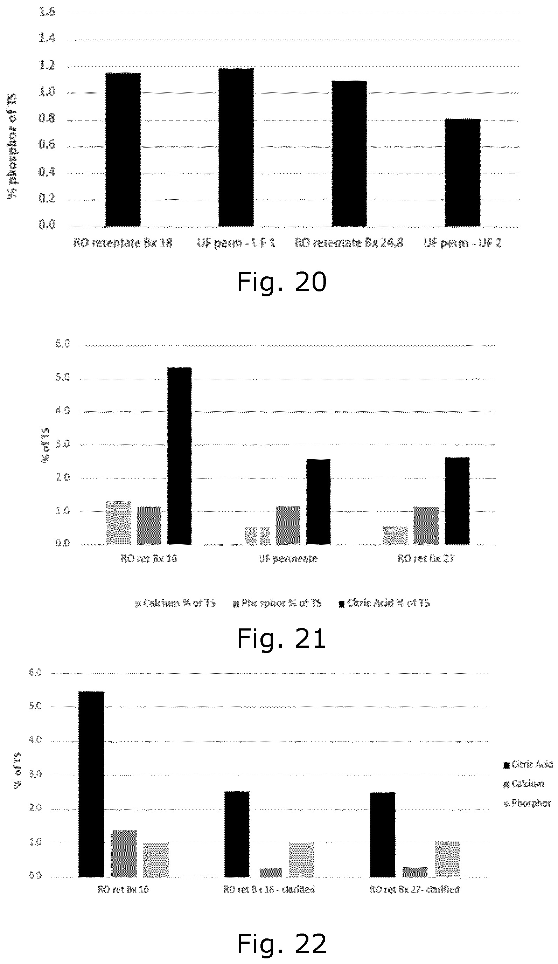

In figure 18, the calcium content in the samples is shown, while figure 19 shows the citric acid content, and figure 20 shows the phosphor content.

As shown in table 6 and figures 18-20, removal of calcium citrate and calcium phosphate can be controlled by varying the pH. Calcium citrate is removed by heating a sample having a pH about 5-6 and calcium phosphate is removed when adjusting the pH to about 7 and heating.

Example 10: Precipitation and separation of calcium citrate by ultrafiltration

The purpose of this example is to show that calcium citrate can be precipitated from a "UF permeate high calcium" in a precipitation step with a concentration to 16°Bx and heating to a temperature of 50°C.

The feed used is a "UF permeate high calcium" obtained by collecting a sample of an ultrafiltration permeate obtained by ultrafiltration of a milk with citric acid added. The "UF permeate high calcium" feed was concentrated by reverse osmosis (RO) to Brix 16 at 7°C.

The precipitation method was carried out as mentioned below:

- The feed "UF permeate high calcium" was concentrated by reverse osmosis (RO) by i) from Brix 5 to Brix 16 and ii) from Brix 5 to Brix 27 at 7°C.
- The RO retentate obtained from i) and ii) was heated to 50°C on a plate heat exchanger.
- The heated RO retentate was kept in a holding tank at 50°C for 60 minutes (time started when the tank was full).
- The precipitate obtained was separated by subjecting the RO retentate to ultrafiltration, 15°C.

The content of calcium, phosphor, citric acid, ash, protein content and the pH were measured using the methods disclosed in example 1. The data are shown in table 7 below and figure 21 based on the total solid content.

Table 7:

|  | RO retentate (Brix 16) | UF permeate | RO retentate (Brix 27) |
|---|---|---|---|
| Ash (% of TS) | 10.22 | 9.99 | 9.17 |
| Calcium (% of TS) | 1.31 | 0.53 | 0.52 |
| Phosphor (% of TS) | 1.14 | 1.16 | 1.15 |
| Protein (% of TS) | 2.99 | 3.13 | 2.59 |
| Citric acid (% of TS) | 5.34 | 2.55 | 2.63 |
| pH | 5.47 | 5.41 | 5.21 |

Table 7 and figure 21 show that the calcium and citric acid levels are reduced significantly when the pH during heating is 5.47. On the contrary, the phosphor content is not reduced, and therefore not precipitated as calcium phosphate at pH 5.47.

In conclusion, calcium citrate precipitates at a pH about 5-6 and can be separated by use of ultrafiltration.

Example 11: Precipitation and separation of calcium citrate by use of clarfier

The purpose of this example is to show that calcium citrate can be precipitated from a "UF permeate high calcium" in a precipitation step with a concentration to Brix 16 and heating to 60°C. Further, the purpose is to show that calcium citrate can be separated by use of a clarifier.

The feed used is a "UF permeate high calcium" obtained by collecting a sample of an ultrafiltration permeate of a milk with citric acid added. The "UF permeate high calcium" feed was concentrated by reverse osmosis (RO) to Brix 16.

The RO membrane used a DOW RO-3838/30-FF and RO was carried out at 35 bar at 8°C.

The precipitation method was carried out as mentioned below:

- The feed "UF permeate high calcium" was concentrated by reverse osmosis (RO) from Brix 5 to Brix 16 and Brix 27, respectively, at 8°C.
- The RO retentate was heated to 40°C on a plate heat exchanger
- When the RO retentate is 40°C, the feed was further heated with direct steam injection to 60°C.
- The heated RO retentate was kept in a holding tank at 60°C for 60 minutes (time started when the tank was full).
- The precipitate was obtained by clarifying the RO retentate in a SPX Seital Separation technology model SE15XXQ3P2 from SPX Flow Technology Santorso s.r.l at 60°C.
- Since some shooting settings at the clarifier were adjusted during the clarification, some precipitated material followed the supernatant. Hence, a second clarification step was conducted.

The content of calcium, phosphor, citric acid content and the pH were measured using the methods disclosed in example 1. The data are shown in table 8 below and figure 22 based on the total solid content.

Table 8:

| | RO retentate (Brix 16) | RO retentate (Brix 16) clarified | RO retentate (Brix 27) clarified |
|---|---|---|---|
| Calcium (% of TS) | 1.38 | 0.27 | 0.28 |
| Phosphor (% of TS) | 1.00 | 1.02 | 1.05 |
| Citric acid (% of TS) | 5.46 | 2.52 | 2.48 |
| pH | 5.57 | 5.56 | |

Table 8 and figure 22 show that the calcium and citric acid levels are reduced significantly when heating having a pH at 5.6. On the contrary, the phosphor content is not reduced, and therefore not precipitated as calcium phosphate at pH 5.6.

In conclusion, calcium citrate precipitates at a pH about 5-6 and can be separated by use of clarification.

Example 12: Precipitation of calcium citrate and removal of calcium citrate by clarifier and ultrafiltration

The purpose of this example is to show that calcium citrate can be precipitated from a "UF permeate high calcium" in a precipitation step with a concentration to Brix 17 and heating to 60°C. Further, the purpose is to show that calcium citrate can be separated by use of a clarifier and ultrafiltration.

The feed used is a "UF permeate high calcium" obtained by collecting a sample of an ultrafiltration permeate of a milk with citric acid added. The "UF permeate high calcium" feed was concentrated by reverse osmosis (RO) to Brix 17.

The RO membrane used a DOW RO-3838/30-FF and RO was carried out at 35 bar at 8°C.

The precipitation method was carried out as mentioned below:

- The feed "UF permeate high calcium" was concentrated by reverse osmosis (RO) from Brix 5 to Brix 17 at 8°C.
- The RO retentate was heated to 40°C on a plate heat exchanger
- The heated RO retentate was kept in a holding tank at 40°C for 60 minutes (time started when the tank was full).
- The precipitate was obtained by clarifying the RO retentate in a clarifier at 60°C. The clarifier used was a SPX Seital Separation technology model SE15XXQ3P2 from SPX Flow Technology Santorso s.r.l.
- Some shooting settings at the clarifier were adjusted during the clarification, some precipitated material followed the supernatant. Therefore, the supernatant was subjected to ultrafiltration.

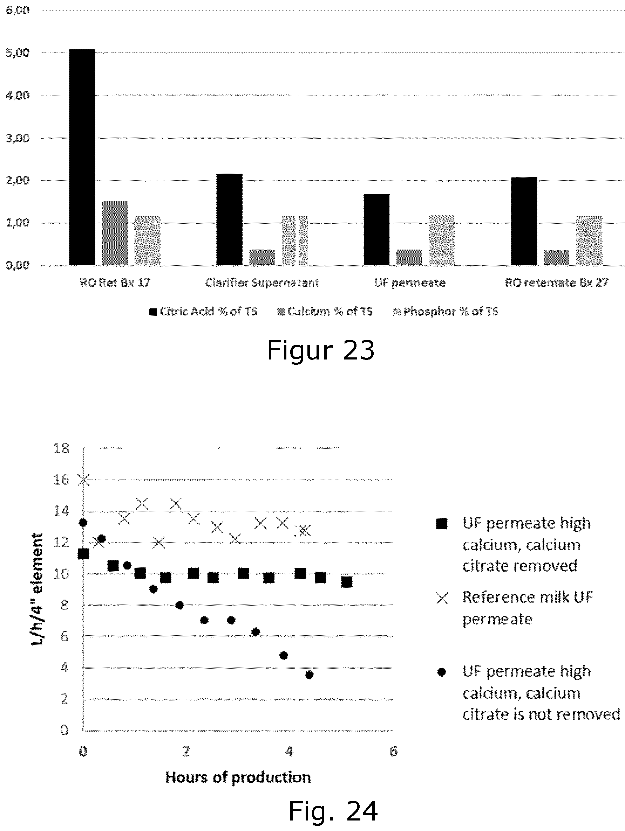

The content of calcium, phosphor, citric acid content and the pH were measured using the methods disclosed in example 1. The data are shown in table 9 below and figure 23 based on the total solid content.

Table 9:

| | RO retentate (Brix 17) | Clarifier supernatant | UF retentate | UF permeate |
|---|---|---|---|---|
| Calcium (% of TS) | 1.52 | 0.37 | 0.43 | 0.38 |
| Phosphor (% of TS) | 1.17 | 1.16 | 1.18 | 1.19 |
| Citric acid (% of TS) | 5.09 | 2.16 | 0.83 | 1.69 |
| pH | 5.47 | 5.38 | 5.38 | 5.39 |

Table 9 and figure 23 show that the calcium and citric acid levels are reduced significantly when heating having a pH at 5.47. On the contrary, the phosphor content is not reduced, and therefore not precipitated as calcium phosphate at pH 5.6.

In conclusion, calcium citrate precipitates at a pH about 5-6 and can be separated by use of clarification and ultrafiltration.

Example 13: Analysis of preheating of "UF permeate high calcium"

The purpose of this example is to show the effect of preheating on the fouling caused by using "UF permeate high calcium" as feed.

The feed used is a "UF permeate high calcium" obtained by collecting a sample of an ultrafiltration permeate obtained by ultrafiltration of a whey with citric acid added.

The feed was heated to 40°C using a plate heat exchanger, and then further heated to 60°C using direct steam.

Calcium citrate was precipitated and after 23 minutes clarification started. Clarification was performed for 40-50 minutes giving an average residence time at 60°C for 47 minutes. The supernatant was further ultrafiltrated to produce a clear product. The clear product was then concentrated by reverse osmosis to Brix 27 using Alfa-Laval HSRO membrane.

The flux data as a measurement of fouling were analysed after concentrating to 27°Bx of:

- The UF permeate high calcium where calcium citrate has been removed by using the method of the present invention
- A reference UF permeate of milk (no citric acid added)
- A UF permeate high calcium where calcium citrate has not been removed.

The flux data are measured as L/h/element where "element" refers to "spiral wound membrane element".

The result is shown in figure 24.

Figure 24 shows that the concentration of the "UF permeate high calcium" where calcium citrate has been removed according to the present invention (squares) is much more stable than the "UF permeate high calcium" where calcium citrate has not been removed (circles). The flux of the "UF permeate high calcium" where calcium citrate has been removed according to the present invention (squares) is a little lower than the reference (crosses).

Hence, this example shows that the invented method of removing calcium citrate from a liquid dairy stream eliminates, or at least reduces, fouling caused by the presence of citric acid during RO filtration.

Example 14: Comparing measurement of °Bx and dry matter:

An example was made to show the difference between the °Bx and dry matter content measured in different samples of milk UF permeate, of UF permeate concentrated by RO and of clarified UF permeate that has been concentrated by RO. The result is shown in table 10 below.

Table 10:

|  | Brix degree (°Bx) | Dry matter (%) | Ratio (%) |
|---|---|---|---|
| UF permeate | | | |
| Sample 1 | 6.0 | 5.55 | 92.50 |
| Sample 2 | 6.7 | 6.15 | 91.79 |
| Sample 3 | 5.8 | 5.38 | 92.76 |
| Sample 4 | 5.9 | 5.46 | 92.54 |
| **Average** | **6.1** | **5.64** | **92.40** |
| UF permeate - concentrated by RO | | | |
| Sample 5 | 18.3 | 17.53 | 95.79 |
| Sample 6 | 16.1 | 15.36 | 95.40 |
| Sample 7 | 16.4 | 15.45 | 94.21 |
| Sample 8 | 17.8 | 16.97 | 95.34 |
| Sample 9 | 17.1 | 16.30 | 95.32 |
| Sample 10 | 17.2 | 16.62 | 96.63 |
| Sample 11 | 17.2 | 16.34 | 95.00 |
| **Average** | **17.16** | **16.37** | **95.38** |
| UF permeate - concentrated by RO and clarified | | | |
| Sample 12 | 16.4 | 15.98 | 97.44 |
| Sample 13 | 15.7 | 15.25 | 97.13 |
| Sample 14 | 15.8 | 15.18 | 96.08 |
| Sample 15 | 15.8 | 15.03 | 95.13 |
| Sample 16 | 15.4 | 15.01 | 97.47 |
| **Average** | **15.82** | **15.29** | **96.65** |

**Claims**

1. A method of removing calcium citrate from a liquid dairy stream, wherein the method comprises the following steps:

> i) providing a liquid dairy stream comprising a) calcium and b) citric acid and/or citrate, and wherein the liquid dairy stream has a pH below 6.2;
> ii) concentrating the liquid dairy stream until the degrees Brix is in the range of 5°Bx to 24°Bx, wherein the concentrating is by using one or more of nanofiltration and reverse osmosis;
> iii) subjecting the concentrated liquid dairy stream to a precipitation step to precipitate calcium citrate, the precipitation step comprises either the precipitation in step A) or the precipitation in step B):
>
>> A) heating the concentrated liquid dairy stream to a temperature of 40°C to 85°C for at least 5 minutes to precipitate calcium citrate;
>> B) seeding of the concentrated liquid dairy stream and storing the seeded concentrated liquid dairy stream at a temperature of 10°C to 40°C;
>
> iv) optionally cooling the heat-treated concentrated liquid dairy stream;
> v) separating precipitated calcium citrate from the heat-treated liquid dairy stream by using one or more devices for mechanical separation of precipitated calcium citrate from the liquid dairy stream.

2. The method according to claim 1, wherein the liquid dairy stream is selected from the group consisting of whey, ultrafiltration permeate, microfiltration permeate and whey protein concentrate.

3. The method according to any of the claims 1 or 2, wherein the pH of the liquid dairy stream after step i) and before step ii) is adjusted to be in the range of 5.5 to 6.1.

4. The method according to any of claims 1 to 3, wherein the heat treatment in step iii) A) is conducted by using one or more selected from the group consisting of heat exchangers, steam infusion and direct steam injection.

5. The method according to claim 4, wherein the heat treatment in step iii) A) is conducted as a two-step heat treatment, where the concentrated dairy product is first heat treated in a heat exchanger to a temperature of up to 50°C, and subsequently heated with direct steam injection or steam infusion to a temperature up to 85°C.

6. The method according to any of claims 1 to 4, wherein the heat treatment in step iii) A) is conducted with direct steam injection or steam infusion to a temperature up to 85°C.

7. The method according to any of the claims 1 to 6, wherein the one or more devices for separation in step v) is one or more selected from the group consisting of a clarifier, a centrifuge, a decanter, a filter and a cyclone.

8. The method according to claim 7, wherein the one or more devices for separation is a combination of a clarifier and ultrafiltration.

9. The method according to any of the claims 1 to 8, wherein the cooling in step iv) is to a temperature in the range of 5°C to 15°C.

10. The method according to any of the claims 1 to 9, wherein the cooling is before or during the separation step v).

11. The method according to any of the claims 1 to 10, wherein the seeding in step iii) B) is selected from the group consisting of direct seeding and indirect seeding.

12. The method according to any of the claims 1 to 11, wherein the liquid dairy stream obtained after separation of calcium citrate in step v) is subjected to a process of removing calcium phosphate, said process comprises the following steps:

> a) concentrating the liquid dairy stream until the degrees Brix is in the range of 15 to 30°Bx;
> b) heating the concentrated liquid dairy stream to a temperature in the range of 40°C to 85°C;
> c) adding an alkaline compound to the concentrated liquid dairy stream to adjust the pH to be at least 6.5 and holding for at least 10 minutes at 40-85°C to precipitate calcium phosphate
> d) optionally cooling the heat-treated concentrated liquid dairy stream;

e) separating precipitated calcium phosphate from the heat-treated liquid dairy stream by using one or more devices for mechanical separation of precipitated calcium phosphate from the liquid dairy stream.

13. A method of preparing crystallized lactose, wherein said method comprises the following steps:

A) providing the liquid dairy stream where calcium citrate has been removed according to any of the claims 1 to 11 or the liquid dairy stream where calcium citrate and calcium phosphate has been removed according to claim 12;
B) adjusting the pH to be in the range of 5.5 to 6.2;
C) concentrating the pH adjusted liquid dairy stream of step B) until the degrees Brix is in the range of 55 to 65°Bx at a temperature of 50°C to 80°C;
D) cooling to a temperature of 5°C to 20°C to obtain crystallized lactose and mother liquor;
E) separating lactose crystals from the mother liquor;
F) optionally washing the lactose crystals with water.

**Patentansprüche**

1. Verfahren zum Entfernen von Calciumcitrat aus einem flüssigen Milchstrom, wobei das Verfahren die folgenden Schritte umfasst:

i) Bereitstellen eines flüssigen Milchstroms, umfassend a) Calcium und b) Citronensäure und/oder Citrat, und wobei der flüssige Milchstrom einen pH-Wert unter 6,2 aufweist;
ii) Konzentrieren des flüssigen Milchstroms, bis der Brix-Wert im Bereich von 5 °Bx bis 24 °Bx liegt, wobei das Konzentrieren unter Verwendung von einem oder mehreren Verfahren aus Nanofiltration und Umkehrosmose erfolgt;
iii) Unterziehen des konzentrierten flüssigen Milchstroms einem Ausfällungsschritt zum Ausfällen von Calciumcitrat, wobei der Ausfällungsschritt entweder die Ausfällung in Schritt A) oder die Ausfällung in Schritt B) umfasst:

A) Erwärmen des konzentrierten flüssigen Milchstroms auf eine Temperatur von 40 °C bis 85 °C für mindestens 5 Minuten, um Calciumcitrat auszufällen;
B) Impfen des konzentrierten flüssigen Milchstroms und Lagern des geimpften konzentrierten flüssigen Milchstroms bei einer Temperatur von 10 °C bis 40 °C;

iv) optionales Abkühlen des wärmebehandelten konzentrierten flüssigen Milchstroms;
v) Abtrennen von ausgefälltem Calciumcitrat von dem wärmebehandelten flüssigen Milchstrom unter Verwendung einer oder mehrerer Vorrichtungen zur mechanischen Abtrennung von ausgefälltem Calciumcitrat von dem flüssigen Milchstrom.

2. Verfahren nach Anspruch 1, wobei der flüssige Milchstrom ausgewählt ist aus der Gruppe bestehend aus Molke, Ultrafiltrationspermeat, Mikrofiltrationspermeat und Molkenproteinkonzentrat.

3. Verfahren nach einem der Ansprüche 1 oder 2, wobei der pH-Wert des flüssigen Milchstroms nach Schritt i) und vor Schritt ii) so eingestellt wird, dass er im Bereich von 5,5 bis 6,1 liegt.

4. Verfahren nach einem der Ansprüche 1 bis 3, wobei die Wärmebehandlung in Schritt iii) A) unter Verwendung von einem oder mehreren durchgeführt wird, die ausgewählt sind aus der Gruppe bestehend aus Wärmetauschern, Dampfinfusion und direkter Dampfinjektion.

5. Verfahren nach Anspruch 4, wobei die Wärmebehandlung in Schritt iii) A) als eine zweistufige Wärmebehandlung durchgeführt wird, bei der das konzentrierte Milchprodukt zunächst in einem Wärmetauscher auf eine Temperatur von bis zu 50 °C wärmebehandelt und anschließend mittels direkter Dampfinjektion oder Dampfinfusion auf eine Temperatur von bis zu 85 °C erhitzt wird.

6. Verfahren nach einem der Ansprüche 1 bis 4, wobei die Wärmebehandlung in Schritt iii) A) mittels direkter Dampfinjektion oder Dampfinfusion auf eine Temperatur von bis zu 85 °C durchgeführt wird.

7. Verfahren nach einem der Ansprüche 1 bis 6, wobei die eine oder mehreren Vorrichtungen zur Abtrennung in Schritt v) eine oder mehrere sind, ausgewählt aus der Gruppe bestehend aus einem Klärapparat, einer Zentrifuge, einem Dekanter, einem Filter und einem Zyklon.

8. Verfahren nach Anspruch 7, wobei die eine oder mehreren Vorrichtungen zur Abtrennung eine Kombination aus einem Klärapparat und Ultrafiltration sind.

9. Verfahren nach einem der Ansprüche 1 bis 8, wobei das Abkühlen in Schritt iv) auf eine Temperatur im Bereich von 5 °C bis 15 °C erfolgt.

10. Verfahren nach einem der Ansprüche 1 bis 9, wobei das Abkühlen vor oder während des Abtrennungsschritts v) erfolgt.

11. Verfahren nach einem der Ansprüche 1 bis 10, wobei das Impfen in Schritt iii) B) ausgewählt ist aus der Gruppe bestehend aus direktem Impfen und indirektem Impfen.

12. Verfahren nach einem der Ansprüche 1 bis 11, wobei der nach Abtrennung von Calciumcitrat in Schritt v) erhaltene flüssige Milchstrom einem Verfahren zum Entfernen von Calciumphosphat unterzogen wird, wobei dieses Verfahren die folgenden Schritte umfasst:

a) Konzentrieren des flüssigen Milchstroms, bis der Brix-Wert im Bereich von 15 bis 30 °Bx liegt;
b) Erwärmen des konzentrierten flüssigen Milchstroms auf eine Temperatur im Bereich von 40 °C bis 85 °C;
c) Zusetzen einer alkalischen Verbindung zu dem konzentrierten flüssigen Milchstrom, um den pH-Wert auf mindestens 6,5 einzustellen, und Halten für mindestens 10 Minuten bei 40-85 °C, um Calciumphosphat auszufällen;
d) optionales Abkühlen des wärmebehandelten konzentrierten flüssigen Milchstroms;
e) Abtrennen von ausgefälltem Calciumphosphat von dem wärmebehandelten flüssigen Milchstrom unter Verwendung einer oder mehrerer Vorrichtungen zur mechanischen Abtrennung von ausgefälltem Calciumphosphat von dem flüssigen Milchstrom.

13. Verfahren zum Herstellen von kristallisierter Lactose, wobei das Verfahren die folgenden Schritte umfasst:

A) Bereitstellen des flüssigen Milchstroms, aus dem Calciumcitrat nach einem der Ansprüche 1 bis 11 entfernt wurde, oder des flüssigen Milchstroms, aus dem Calciumcitrat und Calciumphosphat nach Anspruch 12 entfernt wurden;
B) Einstellen des pH-Werts auf einen Bereich von 5,5 bis 6,2;
C) Konzentrieren des pH-eingestellten flüssigen Milchstroms aus Schritt B), bis der Brix-Wert in einem Bereich von 55 bis 65 °Bx bei einer Temperatur von 50 °C bis 80 °C liegt;
D) Abkühlen auf eine Temperatur von 5 °C bis 20 °C, um kristallisierte Lactose und Mutterlauge zu erhalten;
E) Abtrennen von Lactosekristallen von der Mutterlauge;
F) optionales Waschen der Lactosekristalle mit Wasser.

**Revendications**

1. Procédé d'élimination du citrate de calcium à partir d'un flux laitier liquide, le procédé comprenant les étapes suivantes consistant à :

i) fournir un flux laitier liquide comprenant a) du calcium et b) de l'acide citrique et/ou du citrate, et le flux laitier liquide ayant un pH inférieur à 6,2 ;
ii) concentrer le flux laitier liquide jusqu'à ce que le degré Brix se situe dans la plage de 5°Bx à 24°Bx, la concentration étant effectuée au moyen d'une ou de plusieurs parmi la nanofiltration et l'osmose inverse ;
iii) soumettre le flux laitier liquide concentré à une étape de précipitation pour faire précipiter le citrate de calcium, l'étape de précipitation comprenant soit la précipitation dans l'étape A), soit la précipitation dans l'étape B) :

A) chauffer le flux laitier liquide concentré jusqu'à une température de 40 °C à 85 °C pendant au moins 5 minutes pour faire précipiter le citrate de calcium ;
B) ensemencer le flux laitier liquide concentré et stocker le flux laitier liquide concentré ensemencé, à une température de 10 °C à 40 °C ;

iv) en option refroidir le flux laitier liquide concentré traité thermiquement ;

v) séparer le citrate de calcium précipité d'avec le flux laitier liquide traité thermiquement, en utilisant un ou plusieurs dispositifs de séparation mécanique du citrate de calcium précipité d'avec le flux laitier liquide.

2. Procédé selon la revendication 1, dans lequel le flux laitier liquide est choisi dans le groupe constitué par le lactosérum, le perméat d'ultrafiltration, le perméat de microfiltration et le concentré de protéines de lactosérum.

3. Procédé selon l'une quelconque des revendications 1 et 2, dans lequel le pH du flux laitier liquide après l'étape i) et avant l'étape ii) est ajusté pour se situer dans l'intervalle de 5,5 à 6,1.

4. Procédé selon l'une quelconque des revendications 1 à 3, dans lequel le traitement thermique dans l'étape iii) A) est effectué en utilisant un ou plusieurs moyens choisis dans le groupe constitué par les échangeurs de chaleur, l'infusion de vapeur et l'injection directe de vapeur.

5. Procédé selon la revendication 4, dans lequel le traitement thermique dans l'étape iii) A) est effectué sous forme d'un traitement thermique en deux étapes, le produit laitier concentré étant d'abord traité thermiquement dans un échangeur de chaleur jusqu'à une température de jusqu'à 50 °C, et ensuite chauffé par injection directe de vapeur ou infusion de vapeur jusqu'à une température de jusqu'à 85 °C.

6. Procédé selon l'une quelconque des revendications 1 à 4, dans lequel le traitement thermique dans l'étape iii) A) est effectué par injection directe de vapeur ou infusion de vapeur jusqu'à une température de jusqu'à 85 °C.

7. Procédé selon l'une quelconque des revendications 1 à 6, dans lequel lesdits un ou plusieurs dispositifs de séparation dans l'étape v) consistent en un ou plusieurs dispositifs choisis dans le groupe constitué par un clarificateur, une centrifugeuse, un décanteur, un filtre et un cyclone.

8. Procédé selon la revendication 7, dans lequel lesdits un ou plusieurs dispositifs de séparation est/sont une combinaison d'un clarificateur et d'ultrafiltration.

9. Procédé selon l'une quelconque des revendications 1 à 8, dans lequel le refroidissement dans l'étape iv) s'effectue jusqu'à une température dans la plage de 5 °C à 15 °C.

10. Procédé selon l'une quelconque des revendications 1 à 9, dans lequel le refroidissement est effectué avant ou pendant l'étape de séparation v).

11. Procédé selon l'une quelconque des revendications 1 à 10, dans lequel l'ensemencement dans l'étape iii) B) est choisi dans le groupe constitué par l'ensemencement direct et l'ensemencement indirect.

12. Procédé selon l'une quelconque des revendications 1 à 11, dans lequel le flux laitier liquide obtenu après séparation du citrate de calcium dans l'étape v) est soumis à un processus d'élimination du phosphate de calcium, ledit processus comprend les étapes suivantes consistant à :

a) concentrer le flux laitier liquide jusqu'à ce que le degré Brix se situe dans la plage de 15 à 30°Bx ;
b) chauffer le flux laitier liquide concentré jusqu'à une température dans la plage de 40 °C à 85 °C ;
c) ajouter un composé alcalin au flux laitier liquide concentré pour ajuster le pH afin qu'il soit égal à au moins 6,5 et maintenir pendant au moins 10 minutes à 40-85 °C pour faire précipiter le phosphate de calcium ;
d) en option refroidir le flux laitier liquide concentré traité thermiquement ;
e) séparer le phosphate de calcium précipité d'avec le flux laitier liquide traité thermiquement, en utilisant un ou plusieurs dispositifs de séparation mécanique du phosphate de calcium précipité d'avec le flux laitier liquide.

13. Procédé de préparation de lactose cristallisé, ledit procédé comprenant les étapes suivantes consistant à :

A) fournir le flux laitier liquide duquel le citrate de calcium a été éliminé selon l'une quelconque des revendications 1 à 11 ou le flux laitier liquide duquel le citrate de calcium et le phosphate de calcium ont été éliminés selon la revendication 12 ;
B) ajuster le pH pour qu'il se situe dans l'intervalle de 5,5 à 6,2 ;
C) concentrer le flux laitier liquide à pH ajusté de l'étape B) jusqu'à ce que le degré Brix se situe dans la plage de 55 à 65°Bx à une température de 50 °C à 80 °C ;

D) refroidir jusqu'à une température de 5 °C à 20 °C pour obtenir du lactose cristallisé et de la liqueur mère ;
E) séparer les cristaux de lactose d'avec la liqueur mère ;
F) en option laver les cristaux de lactose avec de l'eau.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Figur 23

Fig. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016135172 A1
- WO 2016193138 A1
- US 4202909 A
- EP 3262200 B1

### Non-patent literature cited in the description

- The content of chloride was measured using potentiometric titration method. ISO 5943/IDF 88. 2006
- *The ash content was measured using the method NMKL*, 2005, vol. 173
- Determination of nitrogen content, Block-digestion method (semi-micro rapid routine method) ISO 8968-3:2007/IDF. *ISO 8968-3:2004(E)/IDF 20-3:2004*, 2007, 20-3
- Determination of nitrogen content. Determination of non-protein-nitrogen content. *ISO 8968-2001/IDF 20-4:2001*
- Milk, cream and evaporated milk. Determination of the total solids content. *ISO 6731:2010E/IDF21:2020E*
- Dried milk, Dried ice-Mixes & Processed Cheese - Determination of lactose content, enzymatic methods, Boehringer Mannheim test-kit. *DS/ISO 5765-2:2002, IDF 79-2*